# EUROPÄISCHE PATENTSCHRIFT

(11) **EP 0 584 625 B1**
(45) Veröffentlichungstag und Bekanntmachung des Hinweises auf die Patenterteilung: **26.03.1997**
(21) Anmeldenummer: 93112732.8
(22) Anmeldetag: 09.08.1993
(51) Int. Cl.: C07D 213/64, A01N 43/40, C07D 401/04, C07D 409/04, C07D 405/04

(54) **Pyridyloxy-acrylsäureester**
Pyridyloxy substituted esters of acrylic acid
Esters de l'acide acrylique substitués par une pyridyloxy

(30) Priorität: 21.08.1992 DE 4227748
(43) Veröffentlichungstag der Anmeldung: 02.03.1994
(73) Patentinhaber: BAYER AG, 51368 Leverkusen (DE)
(72) Erfinder: Lunkenheimer, Winfried, Dr., D-42115 Wuppertal (DE); Assmann, Lutz, Dr., D-23701 Eutin (DE); Dutzmann, Stefan, Dr., D-40721 Hilden (DE); Dehne, Heinz-Wilhelm, Dr., D-40789 Monheim (DE); Hänssler, Gerd, Dr., D-51381 Leverkusen (DE)

(56) Entgegenhaltungen:
- EP-A- 0 090 353
- EP-A- 0 212 859
- EP-A- 0 383 117
- GB-A- 2 238 308

## Beschreibung

Die vorliegende Erfindung betrifft neue Pyridyloxy-acrylsäureester, ein Verfahren zu ihrer Herstellung und ihre Verwendung als Schädlingsbekämpfungsmittel.

Es ist bereits bekannt, daß bestimmte pyridylsubstituierte Acrylsäureester fungizide Eigenschaften besitzen (vgl. DE-OS 3 904 931, GB-2 238 308). So lassen sich z.B. 2-(6-Phenylpyrid-2-yl-thio)-3-methoxy-acrylsäuremethylester, 2-[N-Methyl-N-(6-phenyl-pyrid-2-yl)-amino]-3-methoxy-acrylsäuremethylester zur Bekämpfung von Pilzen einsetzen. Die Wirksamkeit dieser Stoffe ist jedoch insbesondere bei niedrigen Aufwandmengen nicht in allen Anwendungsgebieten völlig zufriedenstellend.

Es wurden nun neue Pyridyloxy-acrylsäureester der Formel 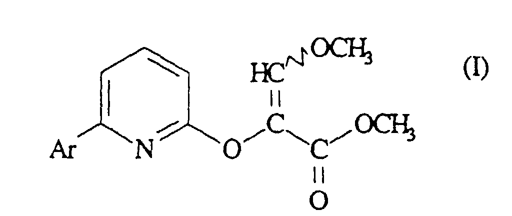 in welcher
- Ar: steht vorzugsweise für Aryl mit 6 bis 10 Kohlenstoffatomen, das einfach oder mehrfach, gleichartig oder verschieden substituiert sein kann durch Halogen, Hydroxy, Cyano, Nitro, geradkettiges oder verzweigtes Alkyl mit 1 bis 6 Kohlenstoffatomen, geradkettiges oder verzweigtes Alkoxy mit 1 bis 6 Kohlenstoffatomen, geradkettiges oder verzweigtes Alkylthio mit 1 bis 6 Kohlenstoffatomen, geradkettiges oder verzweigtes Alkylsulfinyl mit 1 bis 6 Kohlenstoffatomen, geradkettiges oder verzweigtes Alkylsulfonyl mit 1 bis 6 Kohlenstoffatomen, geradkettiges oder verzweigtes Halogenalkyl mit 1 bis 6 Kohlenstoffatomen und 1 bis 13 gleichen oder verschiedenen Halogenatomen, geradkettiges oder verzweigtes Halogenalkoxy mit 1 bis 6 Kohlenstoffatomen und 1 bis 13 gleichen oder verschiedenen Halogenatomen, geradkettiges oder verzweigtes Halogenalkylthio mit 1 bis 6 Kohlenstoffatomen und 1 bis 13 gleichen oder verschiedenen Halogenatomen, geradkettiges oder verzweigtes Halogenalkylsulfinyl mit 1 bis 6 Kohlenstoffatomen und 1 bis 13 gleichen oder verschiedenen Halogenatomen, geradkettiges oder verzweigtes Halogenalkylsulfonyl mit 1 bis 6 Kohlenstoffatomen und 1 bis 13 gleichen oder verschiedenen Halogenatomen, Dialkylamino mit 1 bis 6 Kohlenstoffatomen in jedem Alkylteil, Alkoxycarbonyl mit 1 bis 6 Kohlenstoffatomen im Alkoxyteil, Alkoximinoalkyl mit 1 bis 6 Kohlenstoffatomen im Alkoxyteil und 1 bis 6 Kohlenstoffatomen im Alkylteil, gegebenenfalls einfach bis dreifach, gleichartig oder verschieden durch Halogen, Alkyl mit 1 bis 4 Kohlenstoffatomen und/oder Halogenalkyl mit 1 bis 4 Kohlenstoffatomen und 1 bis 9 gleichen oder verschiedenen Halogenatomen substituiertes, zweifach verknüpftes Alkylen mit 1 bis 6 Kohlenstoffatomen, gegebenenfalls einfach bis dreifach, gleichartig oder verschieden durch Halogen, Alkyl mit 1 bis 4 Kohlenstoffatomen und/oder Halogenalkyl mit 1 bis 4 Kohlenstoffatomen und 1 bis 9 Halogenatomen substituiertes Dioxyalkylen mit 1 bis 6 Kohlenstoffatomen, gegebenenfalls einfach bis dreifach, gleichartig oder verschieden durch Halogen, Alkyl mit 1 bis 4 Kohlenstoffatomen und/oder Halogenalkyl mit 1 bis 4 Kohlenstoffatomen und 1 bis 9 Halogenatomen substituiertes Cycloalkyl mit 3 bis 7 Kohlenstoffatomen, gegebenenfalls einfach bis dreifach, gleichartig oder verschieden durch Halogen, Alkyl mit 1 bis 4 Kohlenstoffatomen und/oder Halogenalkyl mit 1 bis 4 Kohlenstoffatomen und 1 bis 9 Halogenatomen substituiertes Heterocyclyl mit 3 bis 7 Ringgliedem sowie 2 bis 6 Kohlenstoffatomen und 1 bis 3 gleichen oder verschiedenen Heteroatomen, wie Stickstoff, Sauerstoff und/oder Schwefel, und ferner durch Phenyl, Phenoxy, Benzyl, Benzyloxy, Phenylethyl oder Phenylethyloxy, wobei jeder der 6 letztgenannten Substituenten im Phenylteil einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Halogenalkyl mit 1 bis 4 Kohlenstoffatomen und 1 bis 9 gleichen oder verschiedenen Halogenatomen und/oder durch Halogenalkoxy mit 1 bis 4 Kohlenstoffatomen und 1 bis 9 gleichen oder verschiedenen Halogenatomen,
oder
- Ar: steht vorzugsweise für gegebenenfalls benzoannelliertes Heteroaryl mit 2 bis 9 Kohlenstoffatomen und 1 bis 5 gleichen oder verschiedenen Heteroatomen, wie Stickstoff, Sauerstoff und/oder Schwefel, wobei jeder dieser Reste einfach oder mehrfach substituiert sein kann durch Halogen, Hydroxy, Cyano, Nitro, geradkettiges oder verzweigtes Alkyl mit 1 bis 6 Kohlenstoffatomen, geradkettiges oder verzweigtes Alkoxy mit 1 bis 6 Kohlenstoffatomen, geradkettiges oder verzweigtes Alkylthio mit 1 bis 6 Kohlenstoffatomen, geradkettiges oder verzweigtes Alkylsulfinyl mit 1 bis 6 Kohlenstoffatomen, geradkettiges oder verzweigtes Alkylsulfonyl mit 1 bis 6 Kohlenstoffatomen, geradkettiges oder verzweigtes Halogenalkyl mit 1 bis 6 Kohlenstoffatomen und 1 bis 13 gleichen oder verschiedenen Halogenatomen, geradkettiges oder verzweigtes Halogenalkoxy mit 1 bis 6 Kohlenstoffatomen und 1 bis 13 gleichen oder verschiedenen Halogenatomen, geradkettiges oder verzweigtes Halogenalkylthio mit 1 bis 6 Kohlenstoffatomen und 1 bis 13 gleichen oder verschiedenen Halogenatomen, geradkettiges oder verzweigtes Halogenalkylsulfinyl mit 1 bis 6 Kohlenstoffatomen und 1 bis 13 gleichen oder verschiedenen Halogenatomen, geradkettiges oder verzweigtes Halogenalkylsulfonyl mit 1 bis 6 Kohlenstoffatomen und 1 bis 13 gleichen oder verschiedenen Halogenatomen, Dialkylamino mit 1 bis 6 Kohlenstoffatomen in jedem Alkylteil, Alkoxycarbonyl mit 1 bis 6 Kohlenstoffatomen im Alkoxyteil, Alkoximinoalkyl mit 1 bis 6 Kohlenstoffatomen im Alkoxyteil und 1 bis 6 Kohlenstoffatomen im Alkylteil, gegebenenfalls einfach bis dreifach, gleichartig oder verschieden durch Halogen, Alkyl mit 1 bis 4 Kohlenstoffatomen und/oder Halogenalkyl mit 1 bis 4 Kohlenstoffatomen und 1 bis 9 gleichen oder verschiedenen Halogenatomen substituiertes, zweifach verknüpftes Alkylen mit 1 bis 6 Kohlenstoffatomen, gegebenenfalls einfach bis dreifach, gleichartig oder verschieden durch Halogen, Alkyl mit 1 bis 4 Kohlenstoffatomen und/oder Halogenalkyl mit 1 bis 4 Kohlenstoffatomen und 1 bis 9 Halogenatomen substituiertes Dioxyalkylen mit 1 bis 6 Kohlenstoffatomen, gegebenenfalls einfach bis dreifach, gleichartig oder verschieden durch Halogen, Alkyl mit 1 bis 4 Kohlenstoffatomen und/oder Halogenalkyl mit 1 bis 4 Kohlenstoffatomen und 1 bis 9 Halogenatomen substituiertes Cycloalkyl mit 3 bis 7 Kohlenstoffatomen, gegebenenfalls einfach bis dreifach, gleichartig oder verschieden durch Halogen, Alkyl mit 1 bis 4 Kohlenstoffatomen und/oder Halogenalkyl mit 1 bis 4 Kohlenstoffatomen und 1 bis 9 Halogenatomen substituiertes Heterocyclyl mit 3 bis 7 Ringgliedem sowie 2 bis 6 Kohlenstoffatomen und 1 bis 3 gleichen oder verschiedenen Heteroatomen, wie Stickstoff, Sauerstoff und/oder Schwefel, und ferner durch Phenyl, Phenoxy, Benzyl, Benzyloxy, Phenylethyl oder Phenylethyloxy, wobei jeder der 6 letztgenannten Substituenten im Phenylteil einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Halogenalkyl mit 1 bis 4 Kohlenstoffatomen und 1 bis 9 gleichen oder verschiedenen Halogenatomen und/oder durch Halogenalkoxy mit 1 bis 4 Kohlenstoffatomen und 1 bis 9 gleichen oder verschiedenen Halogenatomen,
ausgenommen Verbindungen in denen Ar für unsubstituiertes Phenyl und unsubstituiertes 2-Pyridinyl steht, gefunden.
- Ar: steht besonders bevorzugt für Aryl mit 6 bis 10 Kohlenstoffatomen, das einfach bis fünffach, gleichartig oder verschieden substituiert sein kann durch Halogen, Hydroxy. Cyano, Nitro, geradkettiges oder verzweigtes Alkyl mit 1 bis 4 Kohlenstoffatomen, geradkettiges oder verzweigtes Alkoxy mit 1 bis 4 Kohlenstoffatomen, geradkettiges oder verzweigtes Alkylthio mit 1 bis 4 Kohlenstoffatomen, geradkettiges oder verzweigtes Alkylsulfinyl mit 1 bis 4 Kohlenstoffatomen, geradkettiges oder verzweigtes Alkylsulfonyl mit 1 bis 4 Kohlenstoffatomen, geradkettiges oder verzweigtes Halogenalkyl mit 1 bis 4 Kohlenstoffatomen und 1 bis 9 gleichen oder verschiedenen Halogenatomen, geradkettiges oder verzweigtes Halogenalkoxy mit 1 bis 4 Kohlenstoffatomen und 1 bis 9 gleichen oder verschiedenen Halogenatomen, geradkettiges oder verzweigtes Halogenalkylthio mit 1 bis 4 Kohlenstoffatomen und 1 bis 9 gleichen oder verschiedenen Halogenatomen, geradkettiges oder verzweigtes Halogenalkylsulfinyl mit 1 bis 4 Kohlenstoffatomen und 1 bis 9 gleichen oder verschiedenen Halogenatomen, geradkettiges oder verzweigtes Halogenalkylsulfonyl mit 1 bis 4 Kohlenstoffatomen und 1 bis 9 gleichen oder verschiedenen Halogenatomen, Dialkylamino mit 1 bis 4 Kohlenstoffatomen in jedem Alkylteil, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, gegebenenfalls einfach bis dreifach, gleichartig oder verschieden durch Halogen, Alkyl mit 1 bis 3 Kohlenstoffatomen und/oder Halogenalkyl mit 1 bis 3 Kohlenstoffatomen und 1 bis 7 gleichen oder verschiedenen Halogenatomen substituiertes, zweifach verknüpftes Alkylen mit 1 bis 3 Kohlenstoffatomen, gegebenenfalls einfach bis dreifach, gleichartig oder verschieden durch Halogen, Alkyl mit 1 bis 3 Kohlenstoffatomen und/oder Halogenalkyl mit 1 bis 3 Kohlenstoffatomen und 1 bis 7 Halogenatomen substituiertes Dioxyalkylen mit 1 bis 4 Kohlenstoffatomen, gegebenenfalls einfach bis dreifach, gleichartig oder verschieden durch Halogen, Alkyl mit 1 bis 3 Kohlenstoffatomen und/oder Halogenalkyl mit 1 bis 3 Kohlenstoffatomen und 1 bis 7 Halogenatomen substituiertes Cycloalkyl mit 3 bis 6 Kohlenstoffatomen, gegebenenfalls einfach bis dreifach, gleichartig oder verschieden durch Halogen, Alkyl mit 1 bis 3 Kohlenstoffatomen und/oder Halogenalkyl mit 1 bis 3 Kohlenstoffatomen und 1 bis 7 Halogenatomen substituiertes, gesättigtes Heterocyclyl mit 5 bis 7 Ringgliedern sowie 4 bis 6 Kohlenstoffatomen und 1 oder 2 gleichen oder verschiedenen Heteroatomen, wie Stickstoff, Sauerstoff und/oder Schwefel, und ferner durch Phenyl, Phenoxy, Benzyl, Benzyloxy, Phenylethyl oder Phenylethyloxy, wobei jeder der 6 letztgenannten Substituenten im Phenylteil einfach bis dreifach, gleichartig oder verschieden substituiert sein kann durch Halogen, Alkyl mit 1 bis 3 Kohlenstoffatomen, Alkoxy mit 1 bis 3 Kohlenstoffatomen, Halogenalkyl mit 1 bis 3 Kohlenstoffatomen und 1 bis 7 gleichen oder verschiedenen Halogenatomen und/oder durch Halogenalkoxy mit 1 bis 3 Kohlenstoffatomen und 1 bis 7 gleichen oder verschiedenen Halogenatomen,
oder
- Ar: steht besonders bevorzugt für gegebenenfalls benzoannelliertes Heteroaryl mit 2 bis 9 Kohlenstoffatomen und 1 bis 3 gleichen odr verschiedenen Heteroatomen, wie Stickstoff, Sauerstoff und/oder Schwefel, wobei jeder dieser Reste einfach bis fünffach substituiert sein kann durch Halogen, Hydroxy, Cyano, Nitro, geradkettiges oder verzweigtes Alkyl mit 1 bis 4 Kohlenstoffatomen, geradkettiges oder verzweigtes Alkoxy mit 1 bis 4 Kohlenstoffatomen, geradkettiges oder verzweigtes Alkylthio mit 1 bis 4 Kohlenstoffatomen, geradkettiges oder verzweigtes Alkylsulfinyl mit 1 bis 4 Kohlenstoffatomen, geradkettiges oder verzweigtes Alkylsulfonyl mit 1 bis 4 Kohlenstoffatomen, geradkettiges oder verzweigtes Halogenalkyl mit 1 bis 4 Kohlenstoffatomen und 1 bis 9 gleichen oder verschiedenen Halogenatomen, geradkettiges oder verzweigtes Halogenalkoxy mit 1 bis 4 Kohlenstoffatomen und 1 bis 9 gleichen oder verschiedenen Halogenatomen, geradkettiges oder verzweigtes Halogenalkylthio mit 1 bis 4 Kohlenstoffatomen und 1 bis 9 gleichen oder verschiedenen Halogenatomen, geradkettiges oder verzweigtes Halogenalkylsulfinyl mit 1 bis 4 Kohlenstoffatomen und 1 bis 9 gleichen oder verschiedenen Halogenatomen, geradkettiges oder verzweigtes Halogenalkylsulfonyl mit 1 bis 4 Kohlenstoffatomen und 1 bis 9 gleichen oder verschiedenen Halogenatomen, Dialkylamino mit 1 bis 4 Kohlenstoffatomen in jedem Alkylteil, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, gegebenenfalls einfach bis dreifach, gleichartig oder verschieden durch Halogen, Alkyl mit 1 bis 3 Kohlenstoffatomen und/oder Halogenalkyl mit 1 bis 3 Kohlenstoffatomen und 1 bis 7 gleichen oder verschiedenen Halogenatomen substituiertes, zweifach verknüpftes Alkylen mit 1 bis 4 Kohlenstoffatomen, gegebenenfalls einfach bis dreifach, gleichartig oder verschieden durch Halogen, Alkyl mit 1 bis 3 Kohlenstoffatomen und/oder Halogenalkyl mit 1 bis 3 Kohlenstoffatomen und 1 bis 7 Halogenatomen substituiertes Dioxyalkylen mit 1 bis 4 Kohlenstoffatomen, gegebenenfalls einfach bis dreifach, gleichartig oder verschieden durch Halogen, Alkyl mit 1 bis 3 Kohlenstoffatomen und/oder Halogenalkyl mit 1 bis 3 Kohlenstoffatomen und 1 bis 7 Halogenatomen substituiertes Cycloalkyl mit 3 bis 6 Kohlenstoffatomen, gegebenenfalls einfach bis dreifach, gleichartig oder verschieden durch Halogen, Alkyl mit 1 bis 3 Kohlenstoffatomen und/oder Halogenalkyl mit 1 bis 3 Kohlenstoffatomen und 1 bis 7 Halogenatomen substituiertes Heterocyclyl mit 5 bis 7 Ringgliedern sowie 4 bis 6 Kohlenstoffatomen und 1 oder 2 gleichen oder verschiedenen Heteroatomen, wie Stickstoff, Sauerstoff und/oder Schwefel, und ferner durch Phenyl, Phenoxy, Benzyl, Benzyloxy, Phenylethyl oder Phenylethyloxy, wobei jeder der 6 letztgenannten Substituenten im Phenylteil einfach bis dreifach, gleichartig oder verschieden substituiert sein kann durch Halogen, Alkyl mit 1 bis 3 Kohlenstoffatomen, Alkoxy mit 1 bis 3 Kohlenstoffatomen, Halogenalkyl mit 1 bis 3 Kohlenstoffatomen und 1 bis 7 gleichen oder verschiedenen Halogenatomen und/oder durch Halogenalkoxy mit 1 bis 3 Kohlenstoffatomen und 1 bis 7 gleichen oder verschiedenen Halogenatomenatomen.
- Ar: steht ganz besonders bevorzugt für Phenyl oder Naphthyl, wobei jeder dieser Reste einfach bis dreifach, gleichartig oder verschieden substituiert sein kann durch Fluor, Chlor, Brom, Hydroxy, Cyano, Nitro, Methyl, Ethyl, n- oder 1-Propyl, n-, i-, s- oder t-Butyl, Methoxy, Ethoxy, n- oder i-Propoxy, n-, i-, s- oder t-Butoxy, Methylthio, Ethylthio, Methylsulfinyl, Methylsulfonyl, Trifluormethyl, Trifluormethoxy, Trifluormethylthio, Trifluormethylsulfinyl, Trifluormethylsulfonyl, Dimethylamino, Diethylamino, Methoxycarbonyl, Ethoxycarbonyl, Methoximinomethyl, Methoximinoethyl, Ethoximinomethyl, Ethoximinoethyl, Propan-1,3-diyl, Butan-1,4-diyl, Dioxymethylen, Dioxyethylen, Dioxypropylen, Difluordioxymethylen, Tetrafluordioxyethylen, Cyclopropyl, Cyclopentyl, Cyclohexyl, 1-Pyrrolidinyl, 1 -Piperidinyl, 1-Perhydroazepinyl, 4-Morpholinyl, Phenyl, Phenoxy, Benzyl, Benzyloxy, Phenylethyloxy, wobei jeder der 6 letztgenannten Reste im Phenylteil einfach bis dreifach, gleichartig oder verschieden substituiert sein kann durch Fluor, Chlor, Brom, Methyl, Ethyl, Methoxy, Ethoxy, Trifluormethyl und/oder Trifluormethoxy, oder
- Ar: steht ganz besonders bevorzugt für Furanyl, Thienyl, Pyrrolyl, Oxazolyl, Thiazolyl, Isoxazolyl, Isothiazolyl, Pyrazolyl, Imidazolyl, Oxadiazolyl, Thiadiazolyl, Triazolyl, Pyridyl, Pyridazinyl, Pyrimidinyl, Pyrazinyl oder Triazinyl, wobei jeder dieser Reste benzoannelliert sein kann und einfach bis dreifach, gleichartig oder verschieden substituiert sein kann durch Fluor, Chlor, Brom, Hydroxy, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Methylsulfinyl, Methylsulfonyl, Trifluormethyl, Trifluormethoxy, Trifluormethylthio, Trifluormethylsulfinyl, Trifluormethylsulfonyl, Dimethylamino, Diethylamino, Methoxycarbonyl, Ethoxycarbonyl, Methoximinomethyl, Methoximinoethyl, Ethoximinomethyl, Ethoximinoethyl, Propan-1,3-diyl, Butan-1,4-diyl, Dioxymethylen, Dioxyethylen, Dioxypropylen, Difluordioxymethylen, Tetrafluordioxyethylen, Cyclopropyl, Cyclopentyl, Cyclohexyl, 1-Pyrrolidinyl, 1-Piperidinyl, 1-Perhydroazepinyl, 4-Morpholinyl, Phenyl, Phenoxy, Benzyl, Benzyloxy, Phenylethyloxy, wobei jeder der 6 letztgenannten Reste im Phenylteil einfach bis dreifach, gleichartig oder verschieden substituiert sein kann durch Fluor, Chlor, Brom, Methyl, Ethyl, Methoxy, Ethoxy, Trifluormethyl und/oder Trifluormethoxy.

Die Verbindungen der Formel (I) können in Abhängigkeit von der Stellung der Methoxygruppe an der C=C-Doppelbindung in Form von geometrischen Isomeren vorliegen. Die Erfindung betrifft sowohl die Isomerengemische als auch die einzelnen Isomeren.

Weiterhin wurde gefunden,daß man Pyridyloxy-acrylsäureester der Formel (I) erhält, wenn man Acrylsäure-Derivate der Formel 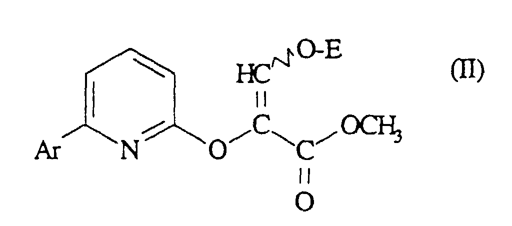 in welcher
- E: für Wasserstoff oder für ein Alkalimetallkation steht und
- Ar: die oben angegebene Bedeutung hat
mit Methylierungsmitteln der Formel

CH₃-A (III)

in welcher
- A: für eine elektronenanziehende Abgangsgruppe steht,
gegebenenfalls in Gegenwart eines Verdünnungsmittels und gegebenenfalls in Gegenwart eines Säurebindemittels sowie gegebenenfalls in Gegenwart eines Katalysators umsetzt.

Schließlich wurde gefunden, daß die neuen Pyridyloxy-acrylsäureester der Formel (I) sehr gut als Schädlingsbekämpfungsmittel geeignet sind. Sie lassen sich insbesondere gegen pflanzenschädigende Mikroorganismen einsetzen.

Überraschenderweise besitzen die erfindungsgemäßen Pyridyloxy-acrylsäureester der Formel (I) gegenüber pflanzenschädigenden Mikroorganismen eine wesentlich bessere Wirksarnkeit als 1-(6-Phenyl-pyrid-2-yl-thio)-2-methoxy-acrylsäuremethylester, 1-[N-Methyl-N-(6-phenyl-pyrid-2-yl)-amino]-2-methoxy-acrylsäuremethylester und 1-[5-(4-Chlorphenyl)-pyrid-3-yl-oxy]-2-methoxy-acrylsäuremethylester, welches strukturell naheliegende, vorbekannte Verbindungen gleicher Wirkungsrichtung sind

Als Beispiele für erfindungsgemäße Stoffe seien die in der folgenden Tabelle aufgeführten Pyridyloxy-acrylsäureester genannt.

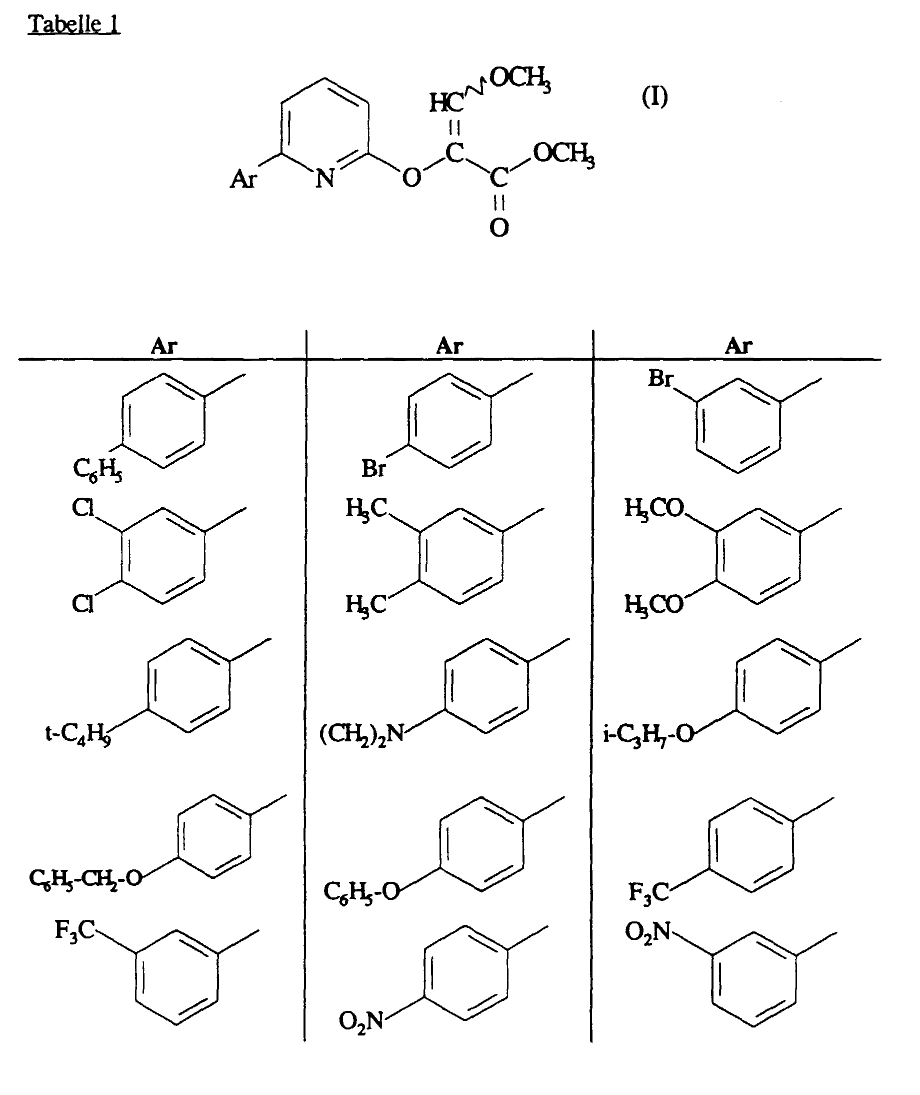

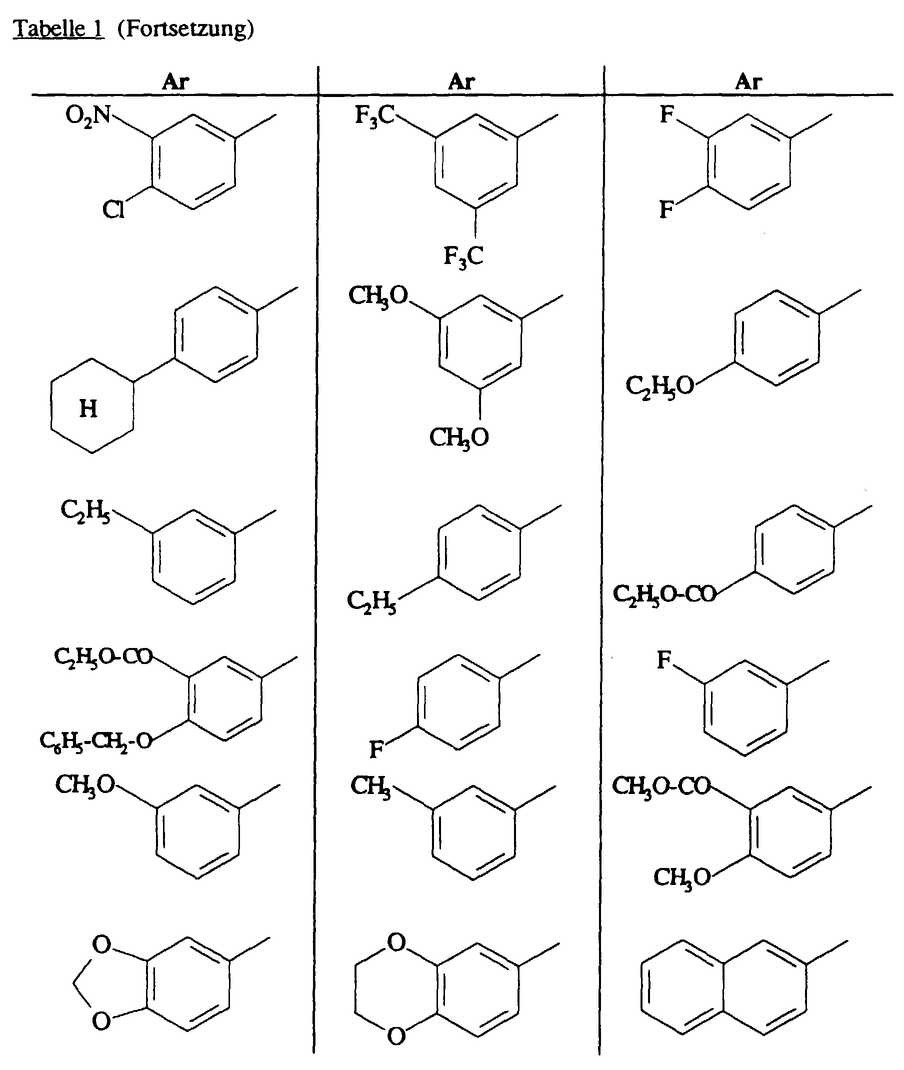

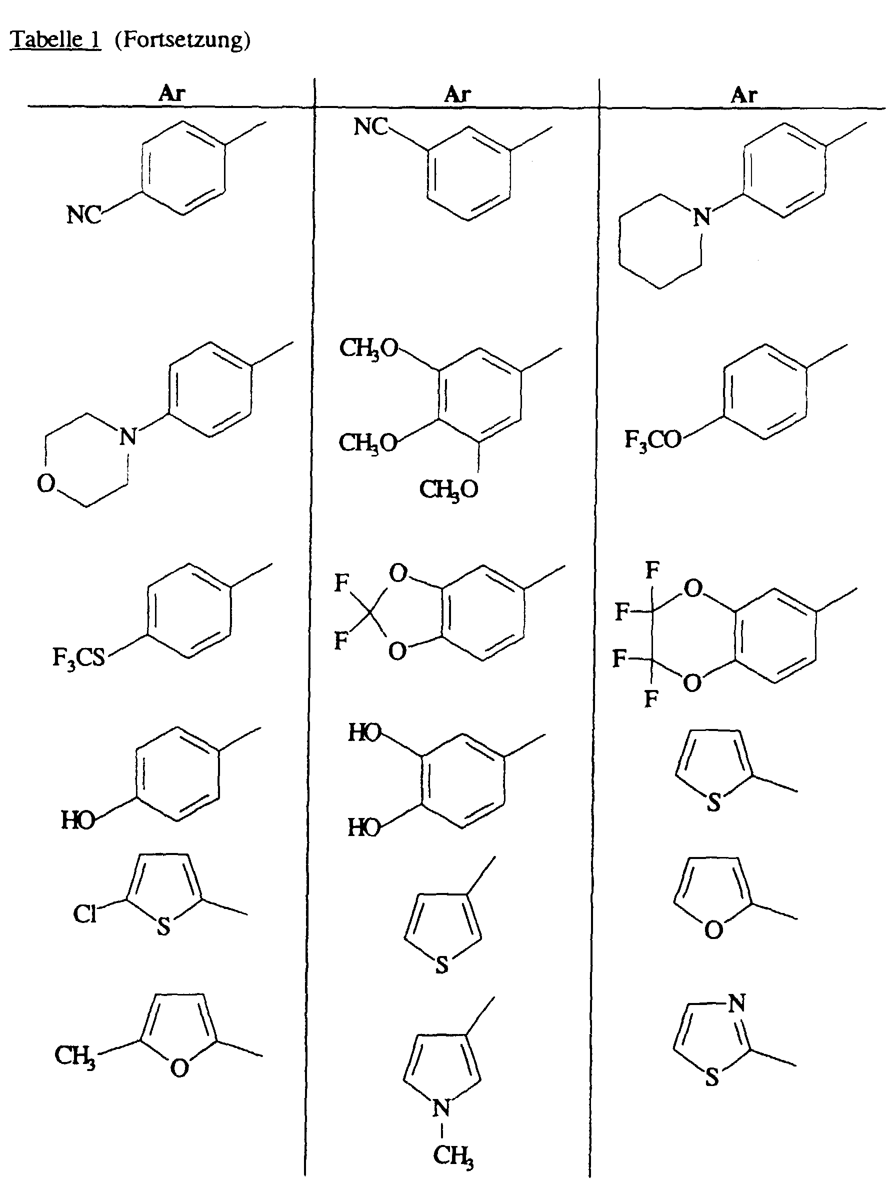

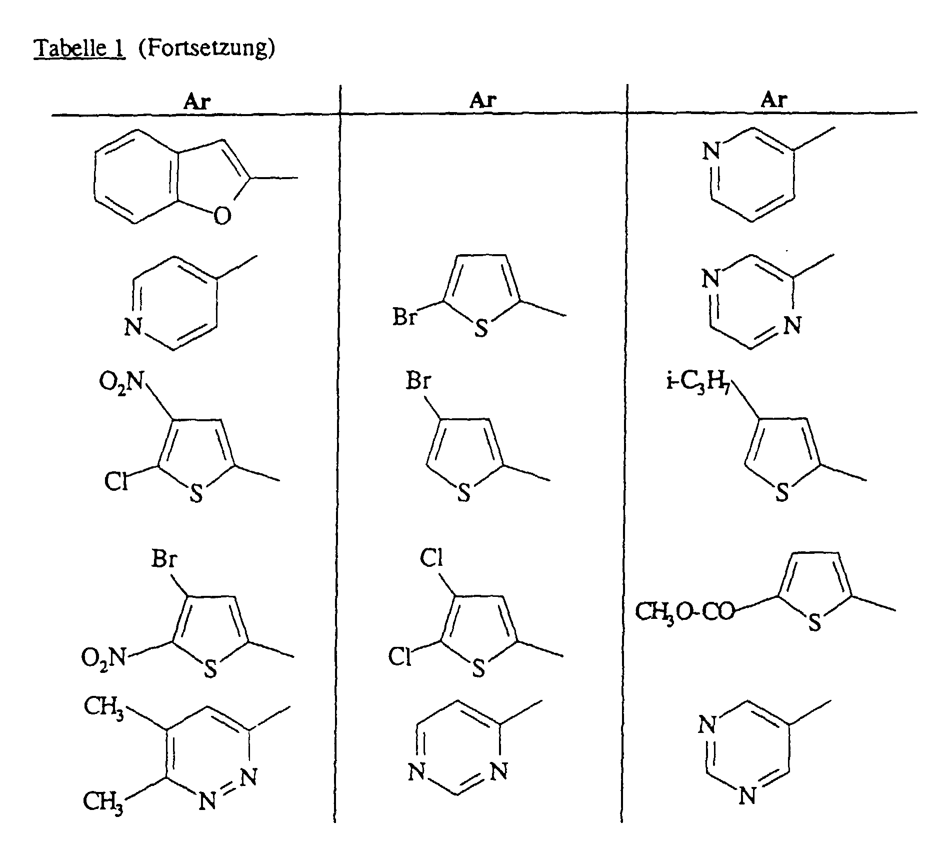

Verwendet man beispielsweise 2-[6-(4-Chlorphenyl)-pyrid-2-yloxy]-1-hydroxymethylen-acrylsäuremethylester und Dimethylsulfat als Ausgangsstoffe, so läßt sich der Reaktionsablauf des erfindungsgemäßen Verfahrens durch das folgende Formelschema darstellen:

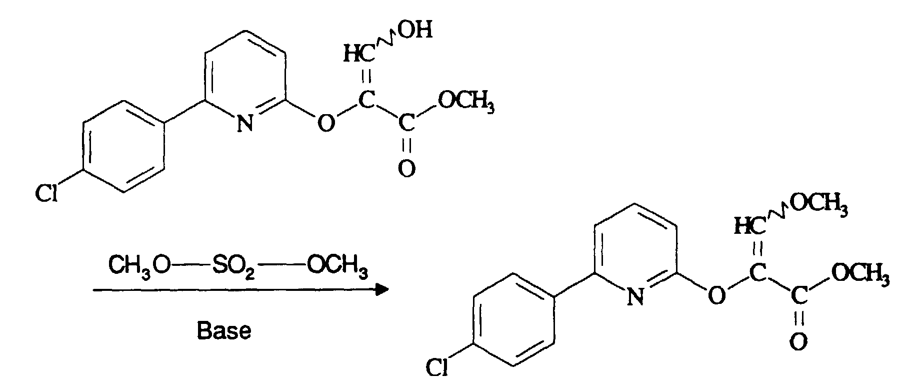

Die bei der Durchführung des erfindungsgemäßen Verfahrens als Ausgangsstoffe benötigten Acrylsäurederivate sind durch die Formel (II) allgemein definiert. In dieser Formel (II) steht Ar vorzugsweise für diejenigen Reste, die bereits im Zusammenhang mit der Beschreibung der erfindungsgemäßen Verbindungen der Formel (I) als bevorzugt für diese Substituenten genannt wurden.
- E: steht vorzugsweise für Wasserstoff oder für ein Natrium- oder Kaliumkation.

Die Acrylsäure-Derivate der Formel (II) sind bisher noch nicht bekannt. Sie lassen sich herstellen, indem man 6-Aryl-2-pyridone der Formel 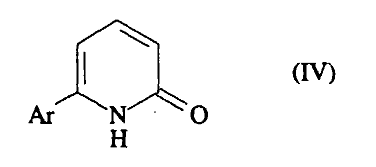 in welcher
- Ar: die oben angegebene Bedeutung hat,
mit Halogenessigsäureestern der Formel

Hal-CH₂-COO-CH₃ (V)

in welcher
- Hal: für Halogen steht,
gegebenenfalls in Gegenwart eines Verdünnungsmittels, und gegebenenfalls in Gegenwart eines Säurebindemittels umsetzt und dann die so erhältlichen Pyridyloxyessigsäureester der Formel (VI), 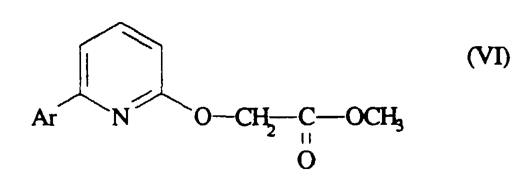 in welcher
- Ar: die oben angegebene Bedeutung hat,
in einer anschließenden zweiten Stufe entweder
a) mit Ameisensäurealkylestern der Formel

   H-COOR (VII)

   in welcher
   - R: für Alkyl steht,
   gegebenenfalls in Gegenwart eines Verdünnungsmittels, oder gegebenenfalls in Gegenwart eines Säurebindemittels umsetzt,
   oder
b) mit Dialkylformamid-Derivate der Formel 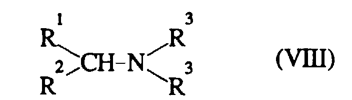 in welcher
   - R¹ und R²: unabhängig voneinander für Alkoxy oder Dialkylamino stehen und
   - R³: für Alkyl steht,
   gegebenenfalls in Gegenwart eines Verdünnungsmittels umsetzt und dann die so erhältlichen 2-Dialkylaminoacrylsäure-Derivate der Formel 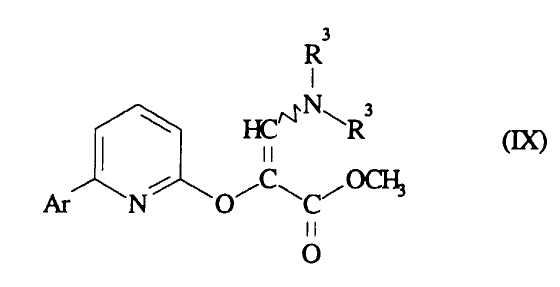 in welcher
   - R³ und Ar: die oben angegebene Bedeutung haben,
   anschließend mit Wasser gegebenenfalls in Gegenwart einer Säure umsetzt,
und die entstehenden Acrylsäure-Derivate, in denen E für Wasserstoff steht, gegebenenfalls mit Hilfe von Alkalimetallhydroxid in die Alkalimetallsalze überführt.

Die bei der Herstellung von Acrylsäure-Derivaten der Formel (II) nach dem obigen Verfahren als Ausgangsstoffe benötigten 6-Aryl-2-pyridone der Formel (IV) sind bekannt oder lassen sich nach prinzipiell bekannten Verfahren herstellen (vergl. z.B. Chem. Ber. 90, 711 [1957]; Ber. dtsch. Chem. Ges. 55, 359 [1922]; Organic Syntheses Coll. Vol. III, 305 [1955]; Angew. Chem. 88, 261, [1976]; Helv. Chim. Acta 24, 233E [1941]).

Die bei dem obigen Verfahren zur Herstellung von Acrylsäure-Derivaten der Formel (II) als Reaktionskomponenten benötigten Halogenessigsäureester sind durch die Formel (V) allgemein definiert In dieser Formel steht Hal vorzugsweise für Chlor, Brom oder Iod. Die Halogenessigsäureester der Formel (V) sind bekannt

Die weiterhin als Reaktionskomponenten benötigten Ameisensäurealkylester sind durch die Formel (VII) allgemein definiert In dieser Formel steht R vorzugsweise für Methyl oder Ethyl. Die Ameisensäurealkylester der Formel (VII) sind bekannt.

Die außerdem als Reaktionskomponenten benötigten Dialkylformamid-Derivate sind durch die Formel (VIII) allgemein definiert In dieser Formel stehen R¹ und R² unabhängig voneinander vorzugsweise für Methoxy, Ethoxy, Dimethylamino oder Diethylamino. R³ steht vorzugsweise für Methyl oder Ethyl. Die Dialkylformamid-Derivate der Formel (VIII) sind bekannt.

Die bei dem obigen Verfahren zur Herstellung von Acrylsäure-Derivaten der Formel (II) als Zwischenprodukte auftretenden Pyridyloxyessigsäureester der Formel (VI) und 2-Dialkylamino-acrylsäure-Derivate der Formel (IX) sind bisher noch nicht bekannt.

Bei der Umsetzung von 6-Aryl-2-pyridonen der Formel (IV) mit Halogenessigsäureestern der Formel (V) kommen als Verdünnungsmittel alle für derartige Reaktionen üblichen, inerten organischen Solventien in Betracht. Vorzugsweise verwendbar sind Ether, wie 1,2-Dimethoxyethan.

Als Säurebindemittel kommen bei der Umsetzung von 6-Aryl-2-pyridonen der Formel (IV) mit Halogenessigsäureestern der Formel (V) alle üblichen starken Basen in Frage. Vorzugsweise verwendbar sind Hydride, wie Natriumhydrid.

Die Reaktionstemperaturen können bei der obigen Umsetzung von 6-Alyl-2-pyridonen der Formel (IV) mit Halogenessigsäureestern der Formel (V) innerhalb eines größeren Bereiches variiert werden. Im allgemeinen arbeitet man bei Temperaturen zwischen 50°C und 150°C.

Bei der Umsetzung von Pyridyloxyessigsäureestern der Formel (VI) mit Ameisensäurealkylestern der Formel (VII) kommen als Verdünnungsmittel alle für derartige Reaktionen üblichen, inerten organischen Solventien in Betracht. Vorzugsweise verwendbar sind Säureamide, wie Dimethylformamid.

Als Säurebindemittel kommen bei der Umsetzung von Pyridyloxyessigsäureestern der Formel (VI) mit Ameisensäurealkylestern der Formel (VII) alle üblichen starken Basen in Frage. Vorzugsweise verwendbar sind Hydride, wie Natriumhydrid.

Die Reaktionstemperaturen können auch bei der Umsetzung von Pyridyloxyessigsäureestern der Formel (VI) mit Ameisensäurealkylestern der Formel (VII) in einem größeren Bereich variiert werden. Im allgemeinen arbeitet man bei Temperaturen zwischen -20°C und +50°C.

Bei der Umsetzung von Pyridyloxyessigsäureestern der Formel (VI) mit Dialkylformamid-Derivaten der Formel (VIII) kommen als Verdünnungsmittel alle für derartige Reaktionen üblichen, inerten, organischen Solventien in Betracht. Vorzugsweise verwendbar sind Ether, wie 1,2-Dimethoxyethan.

Die Reaktionstemperaturen können bei der Umsetzung von Pyridyloxyessigsäureestern der Formel (VI) mit Dialkylformamid-Derivaten der Formel (VIII) ebenfalls innerhalb eines größeren Bereiches variiert werden. Im allgemeinen arbeitet man bei Temperaturen zwischen -20°C und +150°C.

Bei der Umsetzung von 2-Dialkylaminoacrylsäure-Derivaten der Formel (IX) mit Wasser kommen als Säuren alle üblichen starken anorganischen und organischen Säuren in Betracht. Vorzugsweise verwendbar sind wäßrige Halogenwasserstoffsäuren, insbesondere Salzsäure.

Die Reaktionstemperaturen können auch bei der Umsertzung von 2-Dialkylaminoacrylsäure-Derivaten der Formel (IX) mit Wasser innerhalb eines größeren Bereiches variiert werden. Im allgemeinen arbeitet man bei Temperaturen zwischen 0°C und +120°C.

Die Umsetzungen zur Herstellung von Acrylsäure-Derivaten der Formel (II) nach dem obigen Verfahren werden im allgemeinen unter Normaldruck durchgeführt. Es ist jedoch auch möglich, unter erhöhtem oder vermindertem Druck zu arbeiten.

Im übrigen werden die Reaktionskomponenten bei dem obigen Verfahren zur Herstellung von Acrylsäure-Derivaten der Formel (II) je in etwa äquimolaren Mengen eingesetzt. Es ist aber auch möglich, die eine oder andere Komponente in einem Überschuß zu verwenden. Die Aufarbeitung erfolgt jeweils nach üblichen Methoden.

Diejenigen Acrylsäure-Derivate der Formel (II), in denen E für Wasserstoff steht, lassen sich mit Hilfe von Alkalimetallhydroxiden, wie Natriumhydroxid oder Kaliumhydroxid, in die entsprechenden Alkalimetall-Salze überführen.

Die bei der Durchführung des erfindungsgemäßen Verfahrens als Reaktionskomponenten benötigten Methylierungsmittel sind durch die Formel (III) allgemein definiert In dieser Formel steht A vorzugsweise für Halogen, gegebenenfalls substituiertes Alkylsulfonyloxy, gegebenenfalls substituiertes Alkoxysulfonyloxy oder gegebenenfalls substituiertes Arysulfonyloxy. Besonders bevorzugt steht A für Chlor, Brom, Iod, Methylsulfonyloxy, Trifluormethylsulfonyloxy, Methoxysulfonyloxy, Ethoxysulfonyloxy oder p-Tolylsulfonyloxy.

Die Methylierungsmittel der Formel (III) sind allgemein bekannte Verbindungen der organischen Chemie.

Als Verdünnungsmittel zur Durchführung des erfindungsgemäßen Verfahrens kommen inerte organische Lösungsmittel infrage. Vorzugsweise verwendbar sind aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid oder Sulfoxide, wie

Dimethylsulfoxid. Das erfindungsgemäße Verfahren kann gegebenenfalls auch in einem Zweiphasensystem, wie beispielsweise Wasser/Toluol oder Wasser/Dichlormethan, gegebenenfalls in Gegenwart eines geeigneten Phasentransferkatalysators, durchgeführt werden. Als Beispiele für solche Katalysatoren seien genannt: Tetrabutylammoniumiodid, Tetrabutylammoniumbromid, Tetrabutylammoniumchlorid, Tributyl-methylphosphoniumbromid, Trimethyl-C₁₃/C₁₅-alkylammoniumchlorid, Trimethyl-C₁₃/C₁₅-alkylammoniumbromid, Dibenzyl-dimethyl-ammoniummethylsulfat, Dimethyl-C₁₂/C₁₄-alkyl-benzylammoniumchlorid, Dimethyl-C₁₂/C₁₄-alkyl-benzylammoniumbromid, ammoniumhydroxid, Triethylbenzylammoniumchlorid, Methyltrioctylammoniumchlorid, Trimethylbenzylammoniumchiorid, 15-Krone-5, 18-Krone-6 oder Tris-[2-(2-methoxyethoxy)-ethyl]-amin.

Das erfindungsgemäße Verfahren wird vorzugsweise in Gegenwart eines Säurebindemittels durchgeführt. Als solche kommen alle üblichen anorganischen oder organischen Basen infrage. Vorzusweise verwendbar sind Erdalkali- oder Alkalimetallhydride, -hydroxide, -amide, -alkoholate, -acetate, -carbonate oder -hydrogencarbonate, wie beispielsweise Natriumhydrid, Natriumamid, Natriummethylat, Natriumethylat, Kalium-tert-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens in einem größeren Bereich variiert werden. Im allgemeinen arbeitet man bei Temperaturen zwischen -30°C und +120°C, vorzugsweise bei Temperaturen zwischen -20°C und +60°C.

Das erfindungsgemäße Verfahren wird üblicherweise unter Normaldruck durchgeführt. Es ist jedoch auch möglich, unter erhöhtem oder vermindertem Druck zu arbeiten.

Zur Durchführung des erfindungsgemäßen Verfahrens setzt man pro Mol an Acrylsäure-Derivat der Formel (II) im allgemeinen 1,0 bis 10,0 Mol, vorzugsweise 1,0 bis 5,0 Mol an Methylierungsmittel der Formel (III) und gegebenenfalls 1,0 bis 5,0 Mol, vorzugsweise 1,0 bis 2,5 Mol an Säurebindemittel ein. Dabei ist es möglich, die zur Durchführung des erfindungsgemäßen Verfahrens als Ausgangsverbindungen benötigten Acrylsäure-Derivate der Formel (II) in einer vorgelagerten Reaktion direkt im Reaktionsgefäß herzustellen und anschließend ohne Isolierung gemäß dem erfindungsgemäßen Verfahren weiter umzusetzten (Eintopf-Variante). Die Reaktionsdurchführung, Aufarbeitung und Isolierung der Reaktionsprodukte erfolgt nach bekannten Verfahren (vergl. hierzu beispielsweise DE-OS 39 04 931 oder die Herstellungsbeispiele).

Die Reinigung der Endprodukte der Formel (I) erfolgt mit Hilfe üblicher Verfahren, beispielsweise durch Säulenchromatographie oder durch Umkristallisieren.

Die Charakterisierung erfolgt mit Hilfe des Schmelzpunktes oder bei nicht kristallisierenden Verbindungen mit Hilfe des Brechungsindex oder der Protonen-Kernresonanzspektroskopie (¹H-NMR).

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Schädlingsbekämpfungsmittel im Pflanzenschutz, insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes,Deuteromycetes.

Beispielhaft aber nicht begrenzend seien einige Erreger von pilzlichen Krankheiten, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder Peronospora brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielweise Pyrenophora teres oder Pyrenophora graminea (Konidienform: Drechslera, Synonym: Helminthosporium);
Cochliobolus-Arten, wie beispielsweise Cochliobolus sativus (Konidienform: Drechslera, Synonym: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Dabei können die erfindungsgemäßen Wirkstoffe mit besonders gutem Erfolg zur Bekämpfung von Getreidekrankheiten, wie beispielsweise gegen den Erreger des echten Getreidemehltaues an Weizen oder Gerste (Erysiphe graminis) oder gegen den Erreger der Netzfleckenkränkheit der Gerste (Pyrenophora teres) oder gegen den Erreger der Braunfleckenkrankheit an Gerste oder Weizen (Cochliobolus sativus) oder gegen den Erreger der Braunspelzigkeit des Weizens (Leptosphaeria nodorum) oder gegen Erreger von Fusariosen (Fusarium-Arten) oder zur Bekämpfung von Krankheiten im Obst- und Gemüseanbau, wie beispielsweise gegen den Erreger der Tomatenbraunfäule (Phytophthora infestans) oder gegen den Erreger des Apfelschorfes (Venturia inaequalis) oder gegen echte Mehltauarten im Obst- und Gemüseanbau oder zur Bekämpfung von Reiskrankheiten, wie beispielsweise gegen den Erreger der Reisflekkenkrankheit (Pyricularia oryzae) oder gegen den Erreger der Reisstengelkrankheit (Pellicularia sasakii) einsetzen. Daneben besitzen die erfindungsgemäßen Wirkstoffe eine breite in vitro-Wirksamkeit.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und -Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, Alkylnaphthaline, chlorierte Aromaten oder chlorierte aliphatische Kohlenwasserstoffe, wie Chlorbenzole, Chlorethylene, oder Methylenchlorid, aliphatische Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid oder Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen infrage: z.B. natürliche Gesteinsmehle, wie Kaoline, Tonerden, Talkum, Kreide, Quartz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen infrage: z.B. nicht ionogene und anionische Emulgatoren, wie Polyoxyethylen-Fettsäureester, Polyoxyethylen-Fettatkohol-Ether, z.B. Alkylarylpolyglykolether, Alkylsulfonate, Alkylsulfate, Arylsulfonate sowie Eiweißhydrolysate; als Dispergiermittel kommen infrage: z.B. Ligninsulfitablaugen und Methylcellulose.

Es können in den Formulierungen Haftmittel wie Carboxymethylcellulose, natürliche und synthetische, pulverige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinenzwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektizide, Akarizide und Herbizide sowie in Mischungen mit Düngemitteln und Wachstumsregulatoren.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden: Sie liegen im allgemeinen zwischen 1 und 0,0001 Gew.-%, vorzugsweise zwischen 0,5 und 0,001 Gew.- %.

Bei der Saatgutbehandlung werden im allgemeinen Wirkstoffmengen von 0,001 bis 50 g je Kilogramm Saatgut, vorzugsweise 0,01 bis 10 g benötigt.

Bei der Behandlung des Bodens sind Wirkstoffkonzentrationen von 0,00001 bis 0,1 Gew.-%, vorzugsweise von 0,0001 bis 0,02 Gew.-% am Wirkungsort erforderlich.

Die Herstellung und die Verwendung der erfindungsgemäßen Wirkstoffe geht aus den folgenden Beispielen hervor.

### Herstellungsbeispiele:

### Beispiel 1:

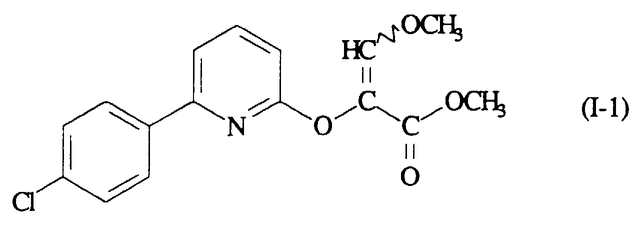

Zu einer Suspension von 0,6 g (0,02 Mol) 80-prozentigem Natriumhydrid in 30 ml absolutem Dimethylformamid gibt man bei 5°C bis 10°C tropfenweise unter Rühren innerhalb von 30 Minuten eine Lösung von 2,9 g (0,01 Mol) 2-[6-(4-Chlorphenyl)-pyrid-2-yloxy]-essigsäuremethylester in 15 g (0,25 Mol) Ameisensäuremethylester und rührt nach beendeter Zugabe 4 Stunden bei Raumtemperatur. Zur Aufarbeitung gibt man unter Eiskühlung 15 ml gesättigte, wässrige Natriumhydrogencarbonatlösung zu, extrahiert dreimal mit jeweils 20 ml Ether, säuert dann die wässrige Phase mit verdünnter Salzsäure an und extrahiert dreimal mit jeweils 20 ml Essigester. Die vereeinigten organischen Phasen werden getrocknet und unter vermindertem Druck eingeengt. Das verbleibende Öl wird in 30 ml absolutem Dimethylformamid gelöst, die Lösung mit 4 g (0,011 Mol) pulverisiertem Kaliumcarbonat versetzt, 15 Minuten bei Raumtemperatur gerührt und anschließend tropfenweise unter Rühren mit 1,3 g (0,011 Mol) Dimethylsulfat versetzt. Danach rührt man 19 Stunden bei Raumtemperatur, gibt dann 20 ml gesättigte wässrige Natriumcarbonatlösung zu, extrahiert dreimal mit Essigester, trocknet die vereinigten organischen Phasen über Natriumsulfat, engt unter vermindertem Druck ein und reinigt den Rückstand durch Chromatographie an Kieselgel (Laufmittel: Dichlormethan).

Man erhält 1,2 g (35% der Theorie) an 2-[6-(4-Chlorphenyl)-pyrid-2-yloxy]-3-methoxy-acrylsäuremethylester vom Schmelzpunkt 50-54°C.

In entsprechender Weise und gemäß den allgemeinen Angaben zur Herstellung erhält man die in der folgenden Tabelle 2 aufgeführten Pyridyloxy-acrylsäureester der Formel (1):

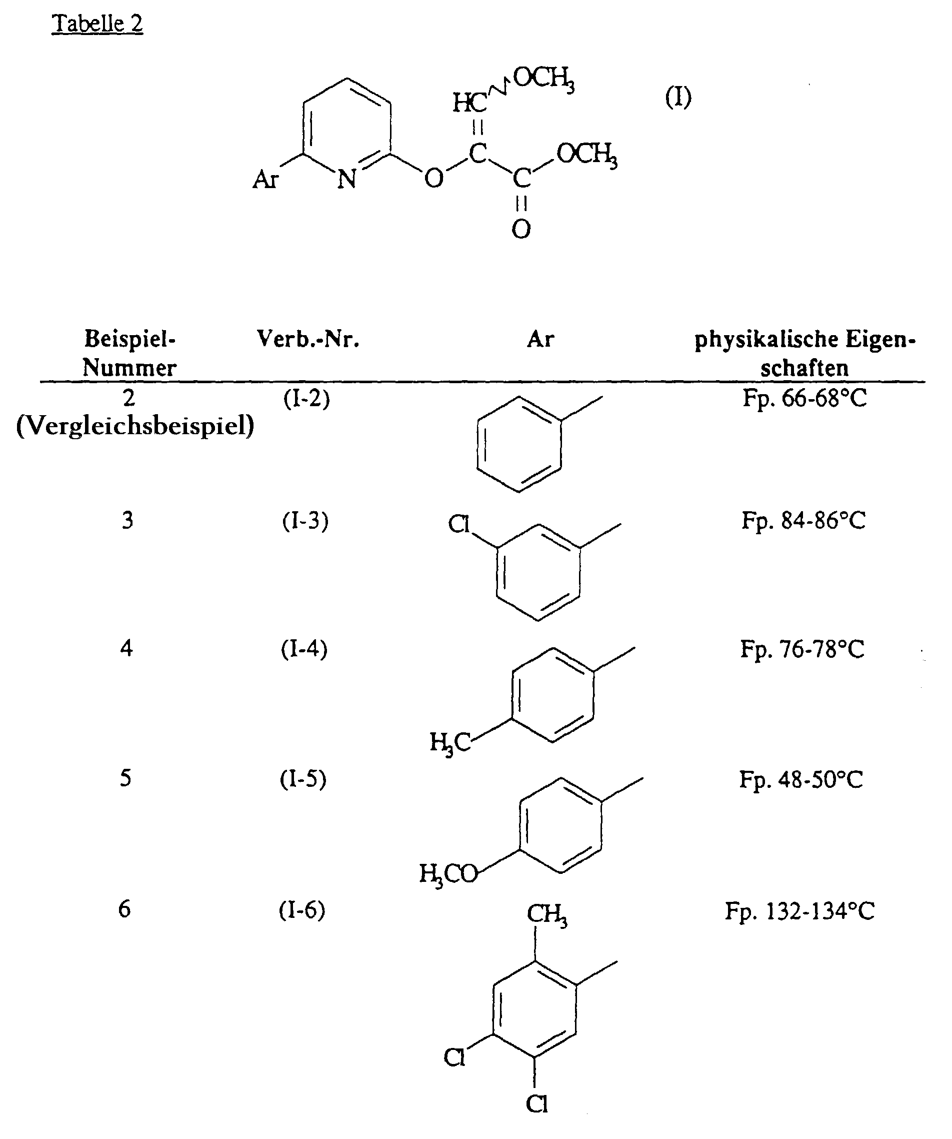

### Herstellung der Ausgangsverbindungen:

### Beispiel 7

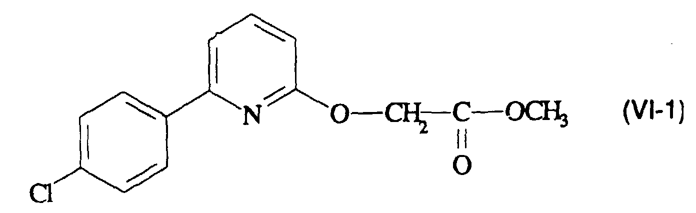

Zu einer Mischung von 3,75 g (0,125 Mol) 80-prozentigem Natriumhydrid, 150 ml 1,2-Dimethoxyethan und 20,8 g (0,1 Mol) 6-(4-Chlorphenyl)-2-pyridon gibt man bei Rückflußtemperatur tropfenweise unter Rühren eine Lösung von 17 g (0,11 Mol) Bromessigsäuremethylester in 25 ml 1,2-Dimethoxyethan und erhitzt anschließend für weitere 4 Stunden auf Rückflußtemperatur. Zur Aufarbeitung wird das Lösungsmittel abdestilliert und der Rückstand zwischen 100 ml Essigester und 30 ml Wasser verteilt Die organische Phase wird abgetrennt, mit Wasser gewaschen, über Natriumsulfat getrocknet und unter vermindertem Druck eingeengt. Der Rückstand wird durch Chromatographie an Kieselgel (Laufmittel: Dichlormethan) gereinigt.

Man erhält 20,3 g (72 % der Theorie) an 2-[6-(4-Chlorphenyl)-pyrid-2-yloxy]-essigsäuremethylester vom Schmelzpunkt 60-63°C.

In entsprechender Weise und gemäß den allgemeinen Angaben zur Herstellung erhält man die in der folgenden Tabelle 3 aufgeführten Pyridyloxyessigsäureester der Formel (VI).

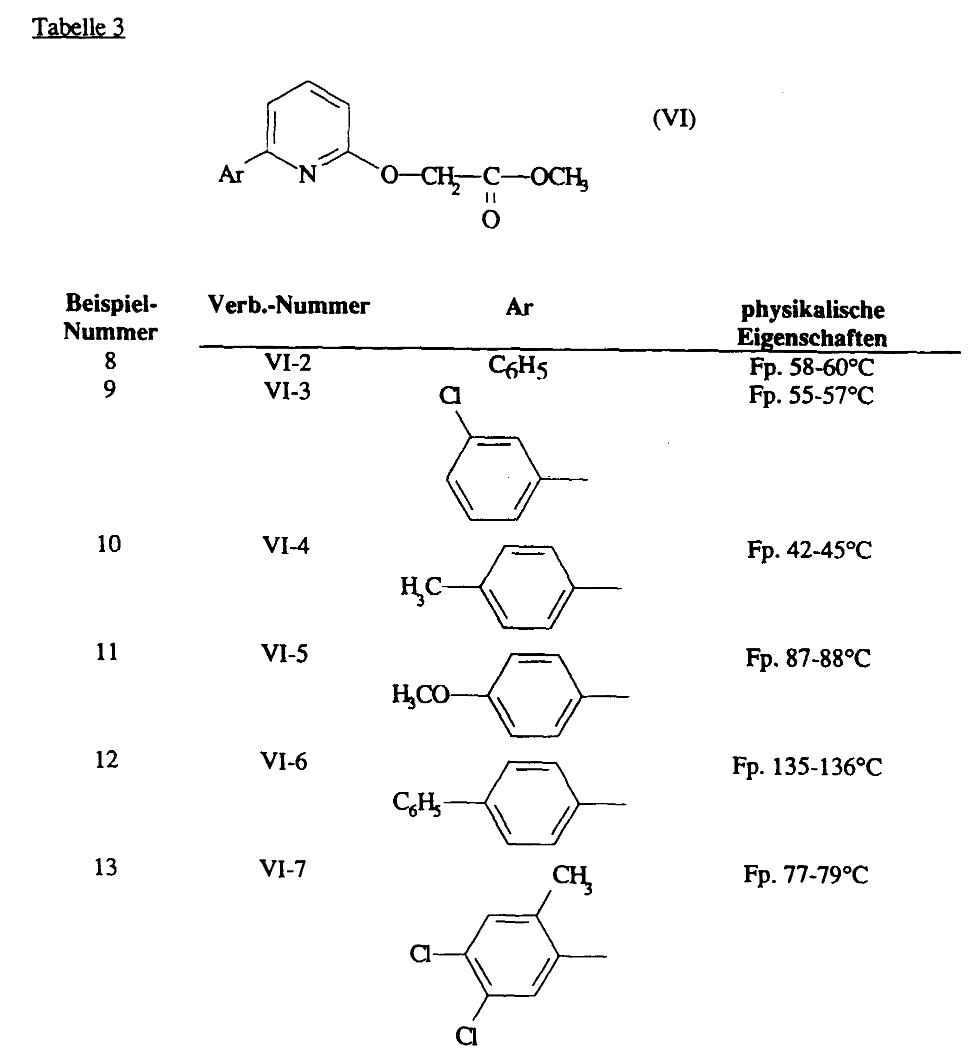

### Beispiel 14

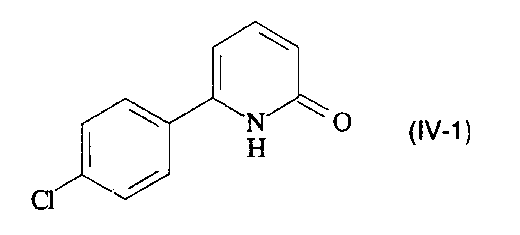

74,2 g (0,35 Mol) 1-(4-Chlorphenyl)-3-dimethylamino-1-propanon (Herstellung vergl. Ber. dtsch. Chem. Ges. 55, 359 [1922]) und 60,2 g (0,35 Mol) 1-Carbamoylmethylpyridiniumchlorid (Herstellung vergl. Helv. Chim. Acta 24, 233E [1941]) in 1,4 l Methanol werden für 3 Stunden auf Rückflußtemperatur erhitzt, wobei gleichzeitig mit einem durch die Reaktionsmischung geleiteten Stickstoffstrom freiwerdendes Dimethylamin ausgetrieben wird. Zur Aufarbeitung wird das Lösungsmittel abdestilliert, der Rückstand mit 700 ml Formamid und 70 ml Eisessig versetzt und für 2 Sunden auf 190°C (Badtemperatur) erhitzt. Zur Aufarbeitung gibt man zu der abgekühlten Reaktionsmischung 650 ml Wasser und extrahiert 5 mal mit jeweils 300 ml Chloroform. Die vereinigten organischen Phasen werden getrocknet und unter vermindertem Druck eingeengt. Der Rückstand wird durch Verrühren mit einem Gemisch aus 300 ml Ether und 30 ml Ethanol zu Kristallisation gebracht, abgesaugt und getrocknet.

Man erhält 23,2 g (32 % der Theorie) an 6-(4-Chlorphenyl)-2-pyridon vom Schmelzpunkt 200-203°C.

In entsprechender Weise und gemäß den allgemeinen Angaben zur Herstellung erhält man die in der folgenden Tabelle 4 aufgeführten 6-Aryl-2-pyridone der Formel (IV).

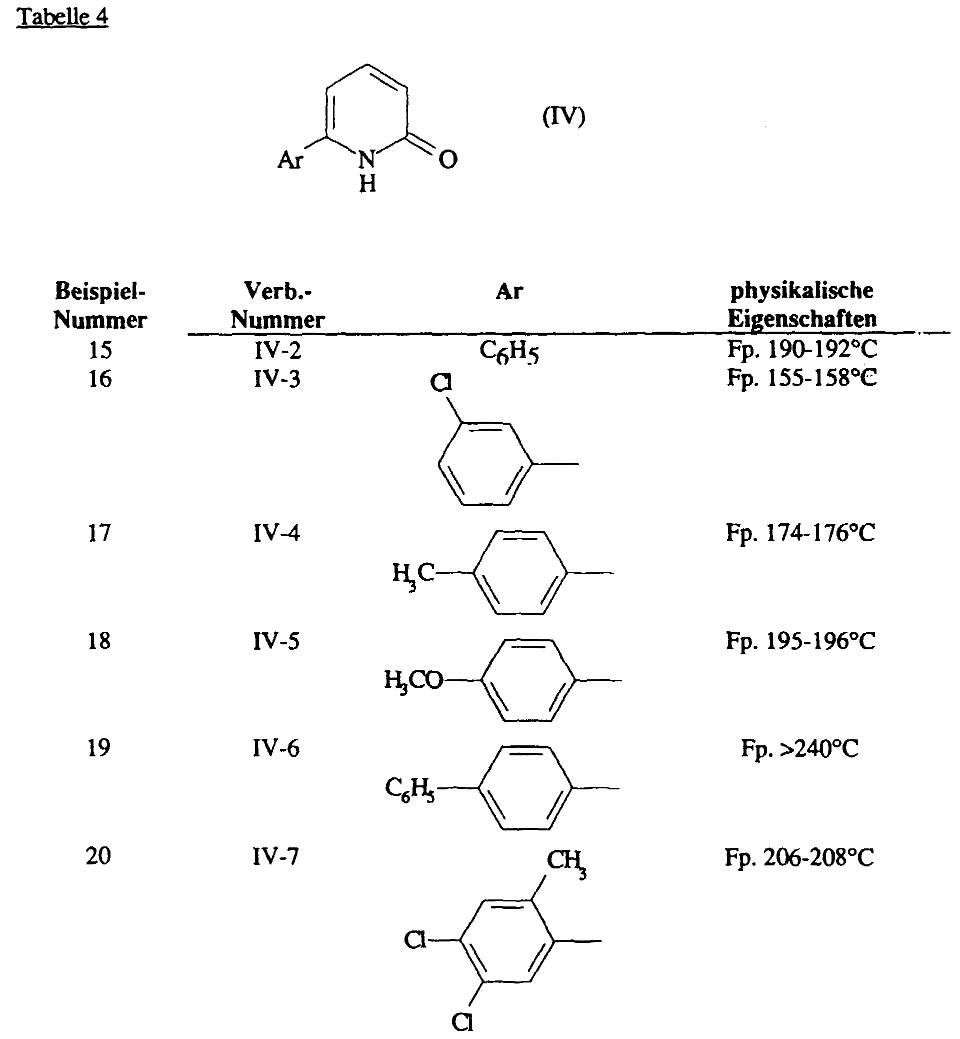

### Anwendungsbeispiele:

In den folgenden Anwendungsbeispielen wurden die nachstehend aufgeführten Verbindungen als Vergleichssubstanzen eingesetzt: 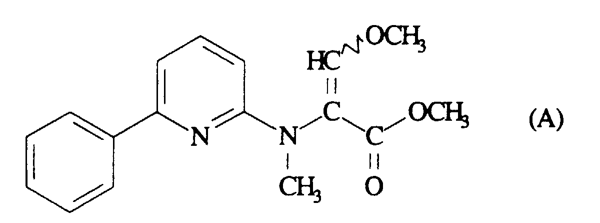 2-[N-Methyl-N-(6-phenyl-pyrid-2-yl)-amino]-3-methoxy-acrylsäuremethylester 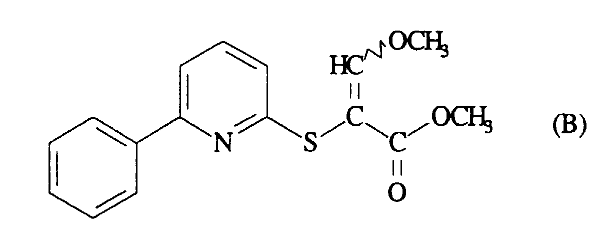 2-(6-Phenyl-pyrid-2-yl-thio)-3-methoxy-acrylsäuremethylester 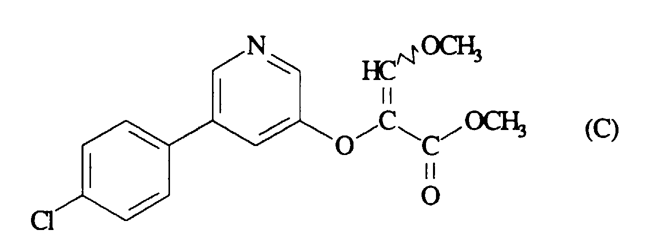 2-[5-(4-Chlorphenyl)-pyrid-3-yl-oxy]-3-methoxy-acrylsäuremethylester
(alle bekannt aus DE-OS 39 04 931)

### Beispiel A:

### Venturia-Test (Apfel) / protektiv

- Lösungsmittel:: 4,7 Gewichtsteile Aceton
- Emulgator:: 0,3 Gewichtsteile Alkyl-Aryl-Polyglykolether

Zur Herstellung einer zweckmäßigen Wirkstoffzubereitung vermischt man ein Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wässrigen Konidiensuspension des Apfelschorferregers (Venturia inaequalis) inokuliert und verbleiben dann einen Tag bei 20°C und 100 % relativer Luftfeuchtigkeit in einer Inkubationskabine.

Die Pflanzen werden dann im Gewächshaus bei 20°C und einer relativen Luftfeuchtigkeit von ca. 70 % aufgestellt.

12 Tage nach der Inokulation erfolgt die Auswertung.

In diesem Test zeigen die in den Beispielen 1 bis 6 aufgeführten erfindungsgemäßen Stoffe bei einer Wirkstoffkonzentration von 10 ppm in der Spritzbrühe, einen Wirkungsgrad von über 80 %, während die Vergleichssubstanz (C) nur einen Wirkungsgrad von etwa 30 % aufweist.

### Beispiel B:

### Leptosphaeria nodorum-Test (Weizen) / protektiv

- Lösungsmittel:: 100 Gewichtsteile Dimethylformamid
- Emulgator:: 0,25 Gewichtsteile Alkyl-Aryl-Polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Sporensuspension von Leptosphaeria nodorum besprüht. Die Pflanzen verbleiben 48 Stunden bei 20°C und 100 % relativer Luftfeuchtigkeit in einer Inkubationskabine.

Die Pflanzen werden in einem Gewächshaus bei einer Temperatur von ca. 15°C und einer relativen Luftfeuchtigkeit von ca. 80 % aufgestellt.

10 Tage nach der Inokulation erfolgt die Auswertung.

In diesem Test zeigen die in den Beispielen 1 und 4 aufgeführten erfindungsgemäßen Stoffe bei einer Wirkstoffkonzentration von 250 ppm in der Spritzbrühe einen Wirkungsgrad von 100 %, während die Vergleichssubstanz (C) keine Wirkung aufweist.

### Beispiel C:

### Erysiphe-Test (Gerste) / protektiv

- Lösungsmittel:: 100 Gewichtsteile Dimethylformamid
- Emulgator:: 0,25 Gewichtsteile Alkyl-Aryl-Polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp. hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

In diesem Test zeigen die in den Beispielen 1 bis 3 aufgeführten erfindungsgemäßen Stoffe bei einer Wirkstoffkonzentration von 250 ppm in der Spritzbrühe einen Wirkungsgrad von über 80 %, während die Vergleichssubstanzen (A) und (B) einen Wirkungsgrad von 25 % aufweisen.

### Beispiel D:

### Phytophthora-Test (Tomate) / protektiv

- Lösungsmittel:: 4,7 Gewichtsteile Aceton
- Emulgator:: 0,3 Gewichtsteile Alkyl-Aryl-Polyglykolether

Zur Herstellung einer zweckmäßigen Wirkstoffzubereitung vermischt man ein Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wässrigen Sporensuspension von Phytophthora infestans inokuliert.

Die Pflanzen werden dann in einer Inkubationskabine bei 20°C und einer relativen Luftfeuchtigkeit von ca. 100 % aufgestellt.

3 Tage nach der Inokulation erfolgt die Auswertung.

In diesem Test zeigen die in den Beispielen 1 bis 4 aufgeführten erfindungsgemäßen Stoffe bei einer Wirkstoffkonzentration von 10 ppm in der Spritzbrühe einen Wirkungsgrad von über 80 %, während die Vergleichssubstanzen (B) und (A) keine Wirkung oder nur einen Wirkungsgrad von 50 % aufweisen.

### Beispiel E:

### Pyricularia Test (Reis) / protektiv

- Lösungsmittel:: 12,5 Gewichtsteile Aceton
- Emulgator:: 0,3 Gewichtsteile Alkyl-Aryl-Polyglykolether

Zur Herstellung einer zweckmäßigen Wirkstoffzubereitung vermischt man ein Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach Antrocknen des Spritzbelages werden die Pflanzen mit einer wässrigen Sporensuspension von Pyricularia oryzae inokuliert

Die Pflanzen werden anschließend im Gewächshaus bei 25°C und einer relativen Luftfeuchtigkeit von 100% aufgestellt

4 Tage nach der Inokulation erfolgt die Auswertung des Krankheitsbefalls.

In diesem Test zeigen die in den Beispielen 1 bis 5 aufgeführten erfindungsgemäßen Stoffe bei einer Wirkstoffkonzentration von 0,025 % in der Spritzbrühe einen Wirkungsgrad von 100 %, während die Vergleichssubstanz (C) nur einen Wirkungsgrad von 30 % aufweist.

### Beispiel F:

### Pellicularia Test (Reis)

- Lösungsmittel:: 12,5 Gewichtsteile Aceton
- Emulgator:: 0,3 Gewichtsteile Alkyl-Aryl-Polyglykolether

Zur Herstellung einer zweckmäßigen Wirkstoffzubereitung vermischt man ein Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf Wirksamkeit bespritzt man junge Reispflanzen im 3 bis 4-Blattstadium mit der Wirkstoffzubereitung bis zur Tropfnässe. Die Pflanzen verbleiben bis zum Abtrocknen im Gewächshaus. Anschließend werden die Pflanzen mit Pellicularia sasakii inokuliert und im Gewächshaus bei 25°C und einer relativen Luftfeuchtigkeit von 100% aufgestellt.

5 bis 8 Tage nach der Inokulation erfolgt die Auswertung des Krankheitsbefalls.

In diesem Test zeigen die in den Beispielen 2, 4 und 5 aufgeführten erfindungsgemäßen Stoffe bei einer Wirkstoffkonzentration von 0,025 % in der Spritzbrühe einen Wirkungsgrad von über 70 % und mehr, während die Vergleichssubstanz (C) einen Wirkungsgrad von 30 % aufweist.

## Patentansprüche

1. Pyridyloxy-acrylsäureester der Formel 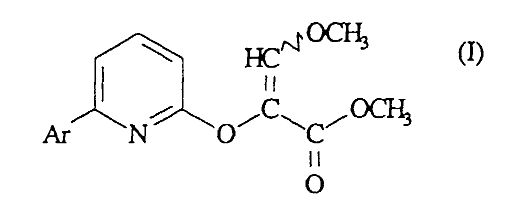 in welcher
Ar für Aryl mit 6 bis 10 Kohlenstoffatomen steht, das einfach oder mehrfach, gleichartig oder verschieden substituiert sein kann durch Halogen, Hydroxy, Cyano, Nitro, geradkettiges oder verzweigtes Alkyl mit 1 bis 6 Kohlenstoffatomen, geradkettiges oder verzweigtes Alkoxy mit 1 bis 6 Kohlenstoffatomen, geradkettiges oder verzweigtes Alkylthio mit 1 bis 6 Kohlenstoffatomen, geradkettiges oder verzweigtes Alkylsulfinyl mit 1 bis 6 Kohlenstoffatomen. geradkettiges oder verzweigtes Alkylsulfonyl mit 1 bis 6 Kohlenstoffatomen, geradkettiges oder verzweigtes Halogenalkyl mit 1 bis 6 Kohlenstoffatomen und 1 bis 13 gleichen oder verschiedenen Halogenatomen, geradkettiges oder verzweigtes Halogenalkoxy mit 1 bis 6 Kohlenstoffatomen und 1 bis 13 gleichen oder verschiedenen Halogenatomen, geradkettiges oder verzweigtes Halogenalkylthio mit 1 bis 6 Kohlenstoffatomen und 1 bis 13 gleichen oder verschiedenen Halogenatomen, geradkettiges oder verzweigtes Halogenalkylsulfinyl mit 1 bis 6 Kohlenstoffatomen und 1 bis 13 gleichen oder verschiedenen Halogenatomen, geradkettiges oder verzweigtes Halogenalkylsulfonyl mit 1 bis 6 Kohlenstoffatomen und 1 bis 13 gleichen oder verschiedenen Halogenatomen, Dialkylamino mit 1 bis 6 Kohlenstoffatomen in jedem Alkylteil, Alkoxycarbonyl mit 1 bis 6 Kohlenstoffatomen im Alkoxyteil, Alkoximinoalkyl mit 1 bis 6 Kohlenstoffatomen im Alkoxyteil und 1 bis 6 Kohlenstoffatomen im Alkylteil, gegebenenfalls einfach bis dreifach, gleichartig oder verschieden durch Halogen, Alkyl mit 1 bis 4 Kohlenstoffatomen und/oder Halogenalkyl mit 1 bis 4 Kohlenstoffatomen und 1 bis 9 gleichen oder verschiedenen Halogenatomen substituiertes, zweifach verknüpftes Alkylen mit 1 bis 6 Kohlenstoffatomen, gegebenenfalls einfach bis dreifach, gleichartig oder verschieden durch Halogen, Alkyl mit 1 bis 4 Kohlenstoffatomen und/oder Halogenalkyl mit 1 bis 4 Kohlenstoffatomen und 1 bis 9 Halogenatomen substituiertes Dioxyalkylen mit 1 bis 6 Kohlenstoffatomen, gegebenenfalls einfach bis dreifach, gleichartig oder verschieden durch Halogen, Alkyl mit 1 bis 4 Kohlenstoffatomen und/oder Halogenalkyl mit 1 bis 4 Kohlenstoffatomen und 1 bis 9 Halogenatomen substituiertes Cycloalkyl mit 3 bis 7 Kohlenstoffatomen, gegebenenfalls einfach bis dreifach, gleichartig oder verschieden durch Halogen, Alkyl mit 1 bis 4 Kohlenstoffatomen und/oder Halogenalkyl mit 1 bis 4 Kohlenstoffatomen und 1 bis 9 Halogenatomen substituiertes Heterocyclyl mit 3 bis 7 Ringgliedem sowie 2 bis 6 Kohlenstoffatomen und 1 bis 3 gleichen oder verschiedenen Heteroatomen, wie Stickstoff, Sauerstoff und/oder Schwefel, und ferner durch Phenyl, Phenoxy, Benzyl, Benzyloxy, Phenylethyl oder Phenylethyloxy, wobei jeder der 6 letztgenannten Substituenten im Phenylteil einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Halogenalkyl mit 1 bis 4 Kohlenstoffatomen und 1 bis 9 gleichen oder verschiedenen Halogenatomen und/oder durch Halogenalkoxy mit 1 bis 4 Kohlenstoffatomen und 1 bis 9 gleichen oder verschiedenen Halogenatomen,
oder
Ar für gegebenenfalls benzoannelliertes Heteroaryl mit 2 bis 9 Kohlenstoffatomen und 1 bis 5 gleichen oder verschiedenen Heteroatomen steht, wobei jeder dieser Reste einfach oder mehrfach substituiert sein kann durch Halogen, Hydroxy, Cyano, Nitro, geradkettiges oder verzweigtes Alkyl mit 1 bis 6 Kohlenstoffatomen, geradkettiges oder verzweigtes Alkoxy mit 1 bis 6 Kohlenstoffatomen, geradkettiges oder verzweigtes Alkylthio mit 1 bis 6 Kohlenstoffatomen, geradkettiges oder verzweigtes Alkylsulfinyl mit 1 bis 6 Kohlenstoffatomen, geradkettiges oder verzweigtes Alkylsulfonyl mit 1 bis 6 Kohlenstoffatomen, geradkettiges oder verzweigtes Halogenalkyl mit 1 bis 6 Kohlenstoffatomen und 1 bis 13 gleichen oder verschiedenen Halogenatomen, geradkettiges oder verzweigtes Halogenalkoxy mit 1 bis 6 Kohlenstoffatomen und 1 bis 13 gleichen oder verschiedenen Halogenatomen, geradkettiges oder verzweigtes Halogenalkylthio mit 1 bis 6 Kohlenstoffatomen und 1 bis 13 -gleichen oder verschiedenen Halogenatomen, geradkettiges oder verzweigtes Halogenalkylsulfinyl mit 1 bis 6 Kohlenstoffatomen und 1 bis 13 gleichen oder verschiedenen Halogenatomen, geradkettiges oder verzweigtes Halogenalkylsulfonyl mit 1 bis 6 Kohlenstoffatomen und 1 bis 13 gleichen oder verschiedenen Halogenatomen, Dialkylamino mit 1 bis 6 Kohlenstoffatomen in jedem Alkylteil, Alkoxycarbonyl mit 1 bis 6 Kohlenstoffatomen im Alkoxyteil, Alkoximinoalkyl mit 1 bis 6 Kohlenstoffatomen im Alkoxyteil und 1 bis 6 Kohlenstoffatomen im Alkylteil, gegebenenfalls einfach bis dreifach, gleichartig oder verschieden durch Halogen, Alkyl mit 1 bis 4 Kohlenstoffatomen und/oder Halogenalkyl mit 1 bis 4 Kohlenstoffatomen und 1 bis 9 gleichen oder verschiedenen Halogenatomen substituiertes, zweifach verknüpftes Alkylen mit 1 bis 6 Kohlenstoffatomen, gegebenenfalls einfach bis dreifach, gleichartig oder verschieden durch Halogen, Alkyl mit 1 bis 4 Kohlenstoffatomen und/oder Halogenalkyl mit 1 bis 4 Kohlenstoffatomen und 1 bis 9 Halogenatomen substituiertes Dioxyalkylen mit 1 bis 6 Kohlenstoffatomen, gegebenenfalls einfach bis dreifach, gleichartig oder verschieden durch Halogen, Alkyl mit 1 bis 4 Kohlenstoffatomen und/oder Halogenalkyl mit 1 bis 4 Kohlenstoffatomen und 1 bis 9 Halogenatomen substituiertes Cycloalkyl mit 3 bis 7 Kohlenstoffatomen, gegebenenfalls einfach bis dreifach, gleichartig oder verschieden durch Halogen, Alkyl mit 1 bis 4 Kohlenstoffatomen und/oder Halogenalkyl mit 1 bis 4 Kohlenstoffatomen und 1 bis 9 Halogenatomen substituiertes Heterocyclyl mit 3 bis 7 Ringgliedem sowie 2 bis 6 Kohlenstoffatomen und 1 bis 3 gleichen oder verschiedenen Heteroatomen, wie Stickstoff, Sauerstoff und/oder Schwefel, und ferner durch Phenyl, Phenoxy, Benzyl, Benzyloxy, Phenylethyl oder Phenylethyloxy, wobei jeder der 6 letztgenannten Substituenten im Phenylteil einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Halogenalkyl mit 1 bis 4 Kohlenstoffatomen und 1 bis 9 gleichen oder verschiedenen Halogenatomen und/oder durch Halogenalkoxy mit 1 bis 4 Kohlenstoffatomen und 1 bis 9 gleichen oder verschiedenen Halogenatomen, ausgenommen Verbindungen in denen Ar für unsubstituiertes Phenyl und unsubstituiertes 2-Pyridinyl steht.

2. Verfahren zur Herstellung von Pyridyloxy-acrylsäureestern der Formel 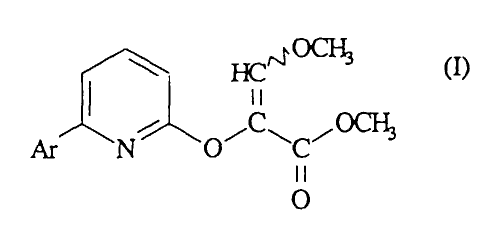 in welcher
Ar die in Anspruch 1 angegebene Bedeutung hat
dadurch gekennzeichnet, daß man Acrylsäure-Derivate der Formel 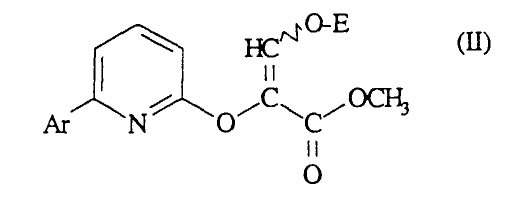 in welcher
E für Wasserstoff oder für ein Alkalimetallkation steht und
Ar die oben angegebene Bedeutung hat
mit Methylierungsmitteln der Formel
CH₃-A (III)
in welcher
A für eine elektronenanziehende Abgangsgruppe steht,
gegebenenfalls in Gegenwart eines Verdünnungsmittels und gegebenenfalls in Gegenwart eines Säurebindemittels sowie gegebenenfalls in Gegenwart eines Katalysators umsetzt.

3. Schädlingsbekämpfungsmittel, gekennzeichnet durch einen Gehalt an mindestens einem Pyridyloxy-acrylsäureester der Formel (I) gemäß Anspruch 1.

4. Verwendung von Pyridyloxy-acrylsäureestrn der Formel (I) gemäß Anspruch 1 zur Bekämpfung von Schädlingen.

5. Verfahren zur Bekämpfung von Schädlingen, dadurch gekennzeichnet, daß man Pyridyloxy-acrylsäureester der Formel (I) gemäß Anspruch 1 auf die Schädlinge und/oder deren Lebensraum ausbringt.

6. Verfahren zur Herstellung von Schädlingsbekämpfungsmitteln, dadurch gekennzeichnet, daß man Pyridyloxy-acrylsäureester der Formel (I) gemäß Anspruch 1 mit Streckmitteln und/oder oberflächenaktiven Stoffen vermischt

7. Acrylsäure-Derivate der Formel 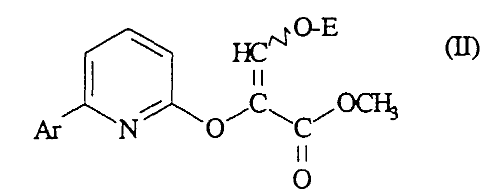 in welcher
Ar die in Anspruch 1 angegebene Bedeutung hat
E für Wasserstoff oder für ein Alkalimetallkation steht

8. Verfahren zur Herstellung von Acrylsäure-Derivaten der Formel 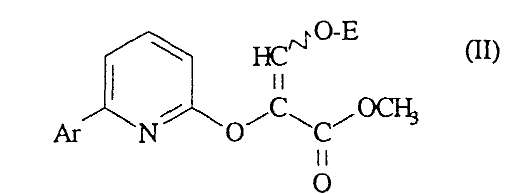 in welcher
Ar die in Anspruch 1 angegebene Bedeutung hat
E für Wasserstoff oder für ein Alkalimetallkation steht,
dadurch gekennzeichnet, daß man in einer ersten Stufe 6-Aryl-2-pyridone der Formel 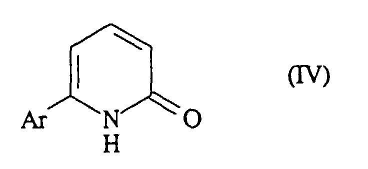 in welcher
Ar die oben angegebene Bedeutung hat,
mit Halogenessigsäureestern der Formel
Hal-CH₂-COO-CH₃ (V)
in welcher
Hal für Halogen steht,
gegebenenfalls in Gegenwart eines Verdünnungsmittels, und gegebenenfalls in Gegenwart eines Säurebindemittels umsetzt und dann die so erhältlichen Pyridyloxyessigsäureester der Formel (VI), 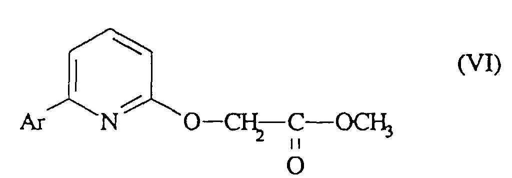 in welcher
Ar die oben angegebene Bedeutung hat,
in einer anschließenden zweiten Stufe entweder
a) mit Ameisensäurealkylestern der Formel
H-COOR (VII)
in welcher
R für Alkyl steht,
gegebenenfalls in Gegenwart eines Verdünnungsmittels, oder gegebenenfalls in Gegenwart eines Säurebindemittels umsetzt,
oder
b) mit Dialkylformamid-Derivaten der Formel 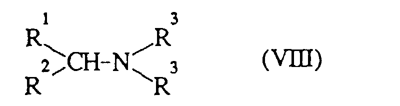 in welcher
R¹ und R² unabhängig voneinander für Alkoxy oder Dialkylamino stehen und
R³ für Alkyl steht,
gegebenenfalls in Gegenwart eines Verdünnungsmittels umsetzt und dann die so erhältlichen 2-Dialkylaminoacrylsäure-Derivate der Formel 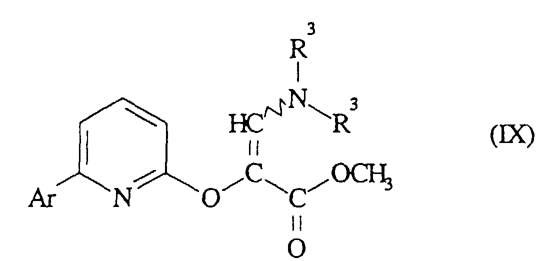 in welcher
R³ und Ar die oben angegebene Bedeutung haben,
anschließend mit Wasser gegebenenfalls in Gegenwart einer Säure umsetzt,
und die entstehenden Acrylsäure-Derivate, in denen E für Wasserstoff steht, gegebenenfalls mit Alkalimetallhydroxiden in Gegenwart eines Verdünnungsmittels umsetzt.

9. Pyridyloxyessigsäureester der Formel (VI), 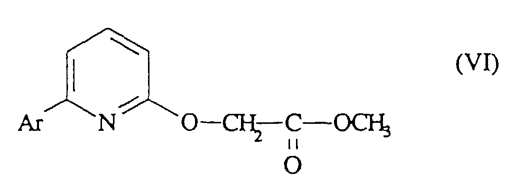 in welcher
Ar die in Anspruch 1 angegebene Bedeutung hat.

10. 2-Dialkylaminoacrylsäure-Derivate der Formel 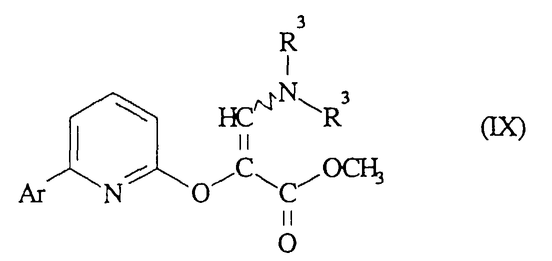 in welcher
Ar die in Anspruch 1 angegebene Bedeutung hat und
R³ für Alkyl steht.

## Claims

1. Pyridyloxy-acrylic acid esters of the formula 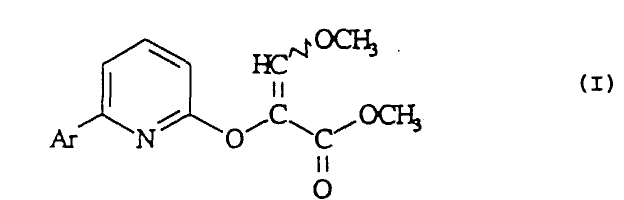 in which
Ar represents aryl having 6 to 10 carbon atoms, which can be substituted identically or differently once or more than once by halogen, hydroxyl, cyano, nitro, straight-chain or branched alkyl having 1 to 6 carbon atoms, straight-chain or branched alkoxy having 1 to 6 carbon atoms, straight-chain or branched alkylthio having 1 to 6 carbon atoms, straight-chain or branched alkylsulphinyl having 1 to 6 carbon atoms, straight-chain or branched alkylsulphonyl having 1 to 6 carbon atoms, straight-chain or branched halogenoalkyl having 1 to 6 carbon atoms and 1 to 13 identical or different halogen atoms, straight-chain or branched halogenoalkoxy having 1 to 6 carbon atoms and 1 to 13 identical or different halogen atoms, straight-chain or branched halogenoalkylthio having 1 to 6 carbon atoms and 1 to 13 identical or different halogen atoms, straight-chain or branched halogenoalkylsulphinyl having 1 to 6 carbon atoms and 1 to 13 identical or different halogen atoms, straight-chain or branched halogenoalkylsulphonyl having 1 to 6 carbon atoms and 1 to 13 identical or different halogen atoms, dialkylamino having 1 to 6 carbon atoms in each alkyl moiety, alkoxycarbonyl having 1 to 6 carbon atoms in the alkoxy moiety, alkoximinoalkyl having 1 to 6 carbon atoms in the alkoxy moiety and 1 to 6 carbon atoms in the alkyl moiety, doubly linked alkylene having 1 to 6 carbon atoms, which is optionally substituted identically or differently once to three times by halogen, alkyl having 1 to 4 carbon atoms and/or halogenoalkyl having 1 to 4 carbon atoms and 1 to 9 identical or different halogen atoms, dioxyalkylene having 1 to 6 carbon atoms, which is optionally substituted identically or differently once to three times by halogen, alkyl having 1 to 4 carbon atoms and/or halogenoalkyl having 1 to 4 carbon atoms and 1 to 9 halogen atoms, cycloalkyl having 3 to 7 carbon atoms, which is optionally substituted identically or differently once to three times by halogen, alkyl having 1 to 4 carbon atoms and/or halogenoalkyl having 1 to 4 carbon atoms and 1 to 9 halogen atoms, heterocyclyl having 3 to 7 ring members as well as 2 to 6 carbon atoms and 1 to 3 identical or different heteroatoms, such as nitrogen, oxygen and/or sulphur, which is optionally substituted identically or differently once to three times by halogen, alkyl having 1 to 4 carbon atoms and/or halogenoalkyl having 1 to 4 carbon atoms and 1 to 9 halogen atoms, and additionally by phenyl, phenoxy, benzyl, benzyloxy, phenylethyl or phenylethyloxy, where each of the 6 latter substituents can be substituted identically or differently once to three times in the phenyl moiety by halogen, alkyl having 1 to 4 carbon atoms, alkoxy having 1 to 4 carbon atoms, halogenoalkyl having 1 to 4 carbon atoms and 1 to 9 identical or different halogen atoms and/or by halogenoalkoxy having 1 to 4 carbon atoms and 1 to 9 identical or different halogen atoms,
or
Ar preferably represents optionally benzo-fused heteroaryl having 2 to 9 carbon atoms and 1 to 5 identical or different heteroatoms, where each of these radicals can be substituted once or more than once by halogen, hydroxyl, cyano, nitro, straight-chain or branched alkyl having 1 to 6 carbon atoms, straight-chain or branched alkoxy having 1 to 6 carbon atoms, straight-chain or branched alkylthio having 1 to 6 carbon atoms, straight-chain or branched alkylsulphinyl having 1 to 6 carbon atoms, straight-chain or branched alkylsulphonyl having 1 to 6 carbon atoms, straight-chain or branched halogenoalkyl having 1 to 6 carbon atoms and 1 to 13 identical or different halogen atoms, straight-chain or branched halogenoalkoxy having 1 to 6 carbon atoms and 1 to 13 identical or different halogen atoms, straight-chain or branched halogenoalkylthio having 1 to 6 carbon atoms and 1 to 13 identical or different halogen atoms, straight-chain or branched halogenoalkylsulphinyl having 1 to 6 carbon atoms and 1 to 13 identical or different halogen atoms, straight-chain or branched halogenoalkylsulphonyl having 1 to 6 carbon atoms and 1 to 13 identical or different halogen atoms, dialkylamino having 1 to 6 carbon atoms in each alkyl moiety, alkoxycarbonyl having 1 to 6 carbon atoms in the alkoxy moiety, alkoximinoalkyl having 1 to 6 carbon atoms in the alkoxy moiety and 1 to 6 carbon atoms in the alkyl moiety, doubly linked alkylene having 1 to 6 carbon atoms, which is optionally substituted identically or differently once to three times by halogen, alkyl having 1 to 4 carbon atoms and/or halogenoalkyl having 1 to 4 carbon atoms and 1 to 9 identical or different halogen atoms, dioxyalkylene having 1 to 6 carbon atoms, which is optionally substituted identically or differently once to three times by halogen, alkyl having 1 to 4 carbon atoms and/or halogenoalkyl having 1 to 4 carbon atoms and 1 to 9 halogen atoms, cycloalkyl having 3 to 7 carbon atoms, which is optionally substituted identically or differently once to three times by halogen, alkyl having 1 to 4 carbon atoms and/or halogenoalkyl having 1 to 4 carbon atoms and 1 to 9 halogen atoms, heterocyclyl having 3 to 7 ring members as well as 2 to 6 carbon atoms and 1 to 3 identical or different heteroatoms, such as nitrogen, oxygen and/or sulphur, which is optionally substituted identically or differently once to three times by halogen, alkyl having 1 to 4 carbon atoms and/or halogenoalkyl having 1 to 4 carbon atoms and 1 to 9 halogen atoms, and additionally by phenyl, phenoxy, benzyl, benzyloxy, phenylethyl or phenylethyloxy, where each of the 6 latter substituents can be substituted in the phenyl moiety identically or differently once to three times by halogen, alkyl having 1 to 4 carbon atoms, alkoxy having 1 to 4 carbon atoms, halogenoalkyl having 1 to 4 carbon atoms and 1 to 9 identical or different halogen atoms and/or by halogenoalkoxy having 1 to 4 carbon atoms and 1 to 9 identical or different halogen atoms, except for compounds in which Ar represents unsubstituted phenyl and unsubstituted 2-pyridinyl.

2. Process for preparing pyridyloxy-acrylic acid esters of the formula 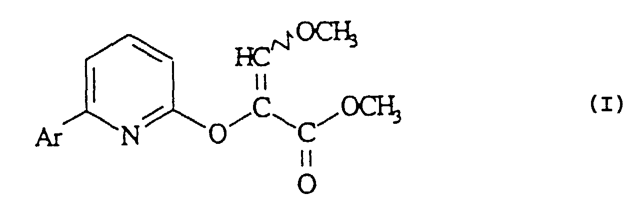 in which
Ar has the meaning given in Claim 1,
characterised in that acrylic acid derivatives of the formula 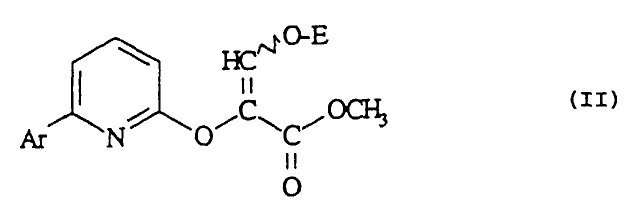 in which
E represents hydrogen or represents an alkali metal cation and
Ar has the abovementioned meaning
are reacted with methylating agents of the formula
CH₃-A (III)
in which
A represents an electron-attracting leaving group, optionally in the presence of a diluent and optionally in the presence of an acid-binding agent as well as optionally in the presence of a catalyst.

3. Agent for combating pests, characterised by a content of at least one pyridyloxy-acrylic acid ester of the formula (I) according to Claim 1.

4. Use of pyridyloxy-acrylic acid esters of the formula (I) according to Claim 1 for combating pests.

5. Process for combating pests, characterised in that pyridyloxy-acrylic acid esters of the formula (I) according to Claim 1 are applied to the pests and/or their habitat.

6. Process for preparing agents for combating pests, characterised in that pyridyloxy-acrylic acid esters of the formula (I) according to Claim 1 are mixed with extenders and/or surface-active compounds.

7. Acrylic acid derivatives of the formula 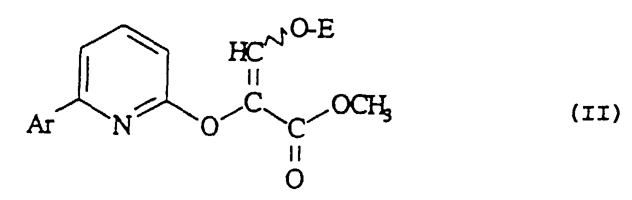 in which
Ar has the meaning given in Claim 1 and
E represents hydrogen or represents an alkali metal cation.

8. Process for preparing acrylic acid derivatives of the formula 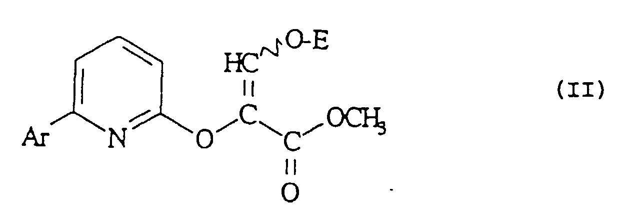 in which
Ar has the meaning given in Claim 1 and
E represents hydrogen or represents an alkali metal cation,
characterised by, in a first stage, reacting 6-aryl-2-pyridones of the formula 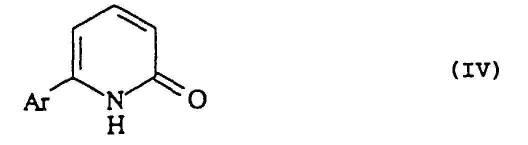 in which
Ar has the abovementioned meaning,
with halogenoacetic acid esters of the formula
Hal-CH₂-COO-CH₃ (V)
in which
Hal represents halogen,
optionally in the presence of a diluent, and optionally in the presence of an acid-binding agent, and then reacting the pyridyloxyacetic acid esters obtainable in this way, of the formula (VI) 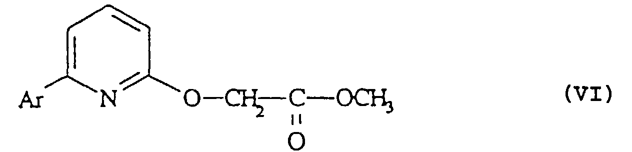 in which
Ar has the abovementioned meaning,
in a subsequent second stage either
a) with alkyl formates of the formula
H-COOR (VII)
in which
R represents alkyl,
optionally in the presence of a diluent, or optionally in the presence of an acid-binding agent,
or
b) with dialkylformamide derivatives of the formula 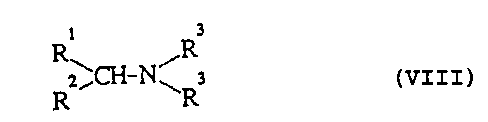 in which
R¹ and R², independently of each other, represent alkoxy or dialkylamino and
R³ represents alkyl,
optionally in the presence of a diluent, and then subsequently reacting the 2-dialkylaminoacrylic acid derivatives obtainable in this way, of the formula 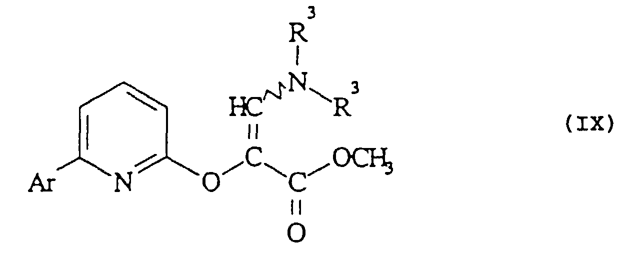 in which
R³ and Ar have the abovementioned meaning,
with water, optionally in the presence of an acid,
and optionally reacting the resulting acrylic acid derivatives, in which E represents hydrogen, with alkali metal hydroxides in the presence of a diluent.

9. Pyridyloxyacetic acid esters of the formula (VI), 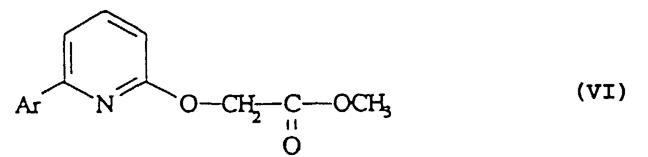 in which
Ar has the meaning given in Claim 1.

10. 2-Dialkylaminoacrylic acid derivatives of the formula 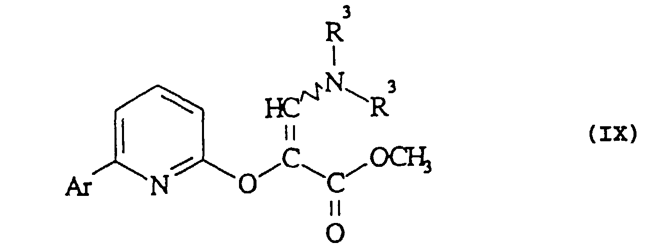 in which
Ar has the meaning given in Claim 1 and
R³ represents alkyl.

## Revendications

1. Esters pyridyloxyacryliques de formule 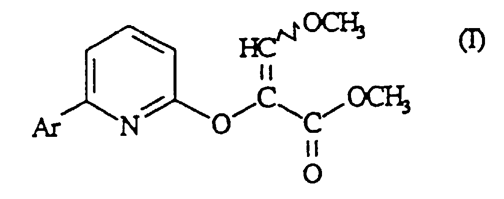 dans laquelle
Ar représente un groupe aryle contenant de 6 à 10 atomes de carbone, qui peut porter un ou plusieurs substituants identiques ou différents halogéno, hydroxyle, cyano, nitro, alkyle à chaîne droite ou ramifiée contenant de 1 à 6 atomes de carbone, alcoxy à chaîne droite ou ramifiée contenant de 1 à 6 atomes de carbone, alkylthio à chaîne droite ou ramifiée contenant de 1 à 6 atomes de carbone, alkylsulfinyle à chaîne droite ou ramifiée contenant de 1 à 6 atomes de carbone, alkylsulfonyle à chaîne droite ou ramifiée contenant de 1 à 6 atomes de carbone, halogénalkyle à chaîne droite ou ramifiée contenant de 1 à 6 atomes de carbone et de 1 à 13 atomes d'halogène identiques ou différents, halogénalcoxy à chaîne droite ou ramifiée contenant de 1 à 6 atomes de carbone et de 1 à 13 atomes d'halogène identiques ou différents, halogénalkylthio à chaîne droite ou ramifiée contenant de 1 à 6 atomes de carbone et de 1 à 13 atomes d'halogène identiques ou différents, halogénalkylsulfinyle à chaîne droite ou ramifiée contenant de 1 à 6 atomes de carbone et de 1 à 13 atomes d'halogène identiques ou différents, halogénalkylsulfonyle à chaîne droite ou ramifiée contenant de 1 à 6 atomes de carbone et de 1 à 13 atomes d'halogène identiques ou différents, dialkylamino contenant de 1 à 6 atomes de carbone dans chaque fraction alkyle, alcoxycarbonyle contenant de 1 à 6 atomes de carbone dans la fraction alcoxy, alcoximinoalkyle contenant de 1 à 6 atomes de carbone dans la fraction alcoxy et de 1 à 6 atomes de carbone dans la fraction alkyle, alkylène deux fois lié contenant de 1 à 6 atomes de carbone, portant éventuellement de 1 à 3 substituants identiques ou différents halogéno, alkyle contenant de 1 à 4 atomes de carbone et/ou halogénalkyle contenant de 1 à 4 atomes de carbone et de 1 à 9 atomes d'halogène identiques ou différents, dioxyalkylène contenant de 1 à 6 atomes de carbone, portant éventuellement de 1 à 3 substituants identiques ou différents halogéno, alkyle contenant de 1 à 4 atomes de carbone et/ou halogénalkyle contenant de 1 à 4 atomes de carbone et de 1 à 9 atomes d'halogène, cycloalkyle contenant de 3 à 7 atomes de carbone, portant éventuellement de 1 à 3 substituants identiques ou différents halogéno, alkyle contenant de 1 à 4 atomes de carbone et/ou halogénalkyle contenant de 1 à 4 atomes de carbone et de 1 à 9 atomes d'halogène, hétérocyclyle contenant de 3 à 7 chaînons cycliques, portant éventuellement de 1 à 3 substituants identiques ou différents halogéno, alkyle contenant de 1 à 4 atomes de carbone et/ou halogénalkyle contenant de 1 à 4 atomes de carbone et de 1 à 9 atomes d'halogène, et contenant de 2 à 6 atomes de carbone et de 1 à 3 hétéroatomes identiques ou différents tels qu'un atome d'azote, un atome d'oxygène et/ou un atome de soufre, ainsi que les substituants phényle, phénoxy, benzyle, benzyloxy, phényléthyle ou phényléthyloxy, chacun des six substituants mentionnés en dernier lieu pouvant porter, dans la fraction phényle, de 1 à 3 substituants identiques ou différents halogéno, alkyle contenant de 1 à 4 atomes de carbone, alcoxy contenant de 1 à 4 atomes de carbone, halogénalkyle contenant de 1 à 4 atomes de carbone et de 1 à 9 atomes d'halogène identiques ou différents, et/ou halogénalcoxy contenant de 1 à 4 atomes de carbone et de 1 à 9 atomes d'halogène identiques ou différents,
ou
Ar représente un groupe hétéroaryle éventuellement benzocondensé contenant de 2 à 9 atomes de carbone et de 1 à 5 hétéroatomes identiques ou différents, chacun de ces radicaux pouvant porter un ou plusieurs substituants identiques ou différents halogéno, hydroxyle, cyano, nitro, alkyle à chaîne droite ou ramifiée contenant de 1 à 6 atomes de carbone, alcoxy à chaîne droite ou ramifiée contenant de 1 à 6 atomes de carbone, alkylthio à chaîne droite ou ramifiée contenant de 1 à 6 atomes de carbone, alkylsulfinyle à chaîne droite ou ramifiée contenant de 1 à 6 atomes de carbone, alkylsulfonyle à chaîne droite ou ramifiée contenant de 1 à 6 atomes de carbone, halogénalkyle à chaîne droite ou ramifiée contenant de 1 à 6 atomes de carbone et de 1 à 13 atomes d'halogène identiques ou différents, halogénalcoxy à chaîne droite ou ramifiée contenant de 1 à 6 atomes de carbone et de 1 à 13 atomes d'halogène identiques ou différents, halogénalkylthio à chaîne droite ou ramifiée contenant de 1 à 6 atomes de carbone et de 1 à 13 atomes d'halogène identiques ou différents, halogénalkylsulfinyle à chaîne droite ou ramifiée contenant de 1 à 6 atomes de carbone et de 1 à 13 atomes d'halogène identiques ou différents, halogénalkylsulfonyle à chaîne droite ou ramifiée contenant de 1 à 6 atomes de carbone et de 1 à 13 atomes d'halogène identiques ou différents, dialkylamino contenant de 1 à 6 atomes de carbone dans chaque fraction alkyle, alcoxycarbonyle contenant de 1 à 6 atomes de carbone dans la fraction alcoxy, alcoximinoalkyle contenant de 1 à 6 atomes de carbone dans la fraction alcoxy et de 1 à 6 atomes de carbone dans la fraction alkyle, alkylène deux fois lié contenant de 1 à 6 atomes de carbone, portant éventuellement de 1 à 3 substituants identiques ou différents halogéno, alkyle contenant de 1 à 4 atomes de carbone et/ou halogénalkyle contenant de 1 à 4 atomes de carbone et de 1 à 9 atomes d'halogène identiques ou différents, dioxyalkylène contenant de 1 à 6 atomes de carbone, portant éventuellement de 1 à 3 substituants identiques ou différents halogéno, alkyle contenant de 1 à 4 atomes de carbone et/ou halogénalkyle contenant de 1 à 4 atomes de carbone et de 1 à 9 atomes d'halogène, cycloalkyle contenant de 3 à 7 atomes de carbone, portant éventuellement de 1 à 3 substituants identiques ou différents halogéno, alkyle contenant de 1 à 4 atomes de carbone et/ou halogénalkyle contenant de 1 à 4 atomes de carbone et de 1 à 9 atomes d'halogène, hétérocyclyle contenant de 3 à 7 chaînons cycliques, portant éventuellement de 1 à 3 substituants identiques ou différents halogéno, alkyle contenant de 1 à 4 atomes de carbone et/ou halogénalkyle contenant de 1 à 4 atomes de carbone et de 1 à 9 atomes d'halogène, et contenant de 2 à 6 atomes de carbone et de 1 à 3 hétéroatomes identiques ou différents tels qu'un atome d'azote, un atome d'oxygène et/ou un atome de soufre, ainsi que les substituants phényle, phénoxy, benzyle, benzyloxy, phényléthyle ou phényléthyloxy, chacun des six substituants mentionnés en dernier lieu pouvant porter, dans la fraction phényle, de 1 à 3 substituants identiques ou différents halogéno, alkyle contenant de 1 à 4 atomes de carbone, alcoxy contenant de 1 à 4 atomes de carbone, halogénalkyle contenant de 1 à 4 atomes de carbone et de 1 à 9 atomes d'halogène identiques ou différents, et/ou halogénalcoxy contenant de 1 à 4 atomes de carbone et de 1 à 9 atomes d'halogène identiques ou différents, à l'exclusion des composés dans lesquels Ar représente un groupe phényle non substitué et un groupe 2-pyridinyle non substitué.

2. Procédé pour la préparation d'esters pyridyloxyacryliques de formule 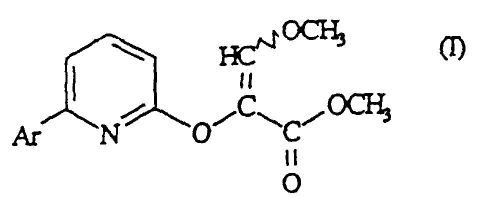 dans laquelle
Ar a la signification indiquée à la revendication 1,
caractérisé en ce qu'on fait réagir des dérivés d'acide acrylique de formule 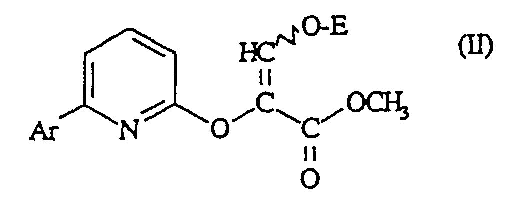 dans laquelle
E représente un atome d'hydrogène ou un cation de métal alcalin et
Ar a la signification indiquée ci-dessus,
avec des agents de méthylation de formule
CH₃-A (III)
dans laquelle
A représente un groupe qui s'éloigne, attirant les électrons,
éventuellement en présence d'un diluant et éventuellement en présence d'un agent neutralisant les acides, ainsi qu'éventuellement en présence d'un catalyseur.

3. Agent de lutte contre les parasites, caractérisé par une teneur en au moins un ester pyridyloxyacrylique de formule (I) selon la revendication 1.

4. Utilisation d'esters pyridyloxyacryliques de formule (I) selon la revendication 1 pour lutter contre les parasites.

5. Procédé pour lutter contre les parasites, caractérisé en ce qu'on répand des esters pyridyloxyacryliques de formule (I) selon la revendication 1, sur les parasites et/ou sur leur biotope.

6. Procédé pour la préparation d'agents de lutte contre les parasites, caractérisé en ce qu'on mélange des esters pyridyloxyacryliques de formule (I) selon la revendication 1, avec des diluants et/ou des matières tensioactives.

7. Dérivés d'acide acrylique de formule 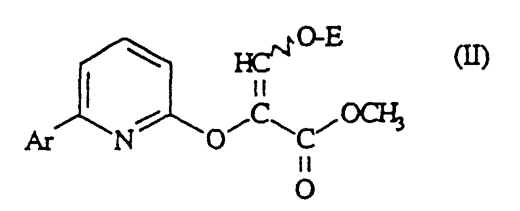 dans laquelle
Ar a la signification indiquée à la revendication 1 et
E représente un atome d'hydrogène ou un cation de métal alcalin.

8. Procédé pour la préparation de dérivés d'acide acrylique de formule 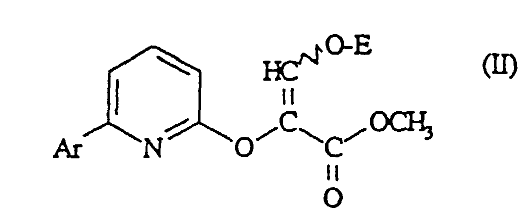 dans laquelle
Ar a la signification indiquée à la revendication 1 et
E représente un atome d'hydrogène ou un cation de métal alcalin,
caractérisé en ce qu'on fait réagir, dans une première étape, des 6-aryl-2-pyridones répondant à la formule 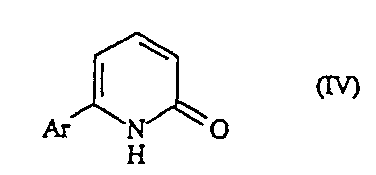 dans laquelle
Ar a la signification indiquée ci-dessus,
avec des esters halogénacétiques de formule
Hal-CH₂-COO-CH₃ (V)
dans laquelle
Hal représente un atome d'halogène,
éventuellement en présence d'un diluant et éventuellement en présence d'un agent neutralisant les acides, puis on fait réagir les esters pyridyloxyacétiques ainsi obtenus répondant à la formule (VI) 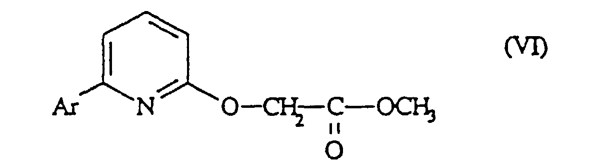 dans laquelle
Ar a la signification indiquée ci-dessus,
dans une deuxième étape ultérieure, soit
a) avec des esters alkyliques d'acide formique de formule
H-COOR (VII)
dans laquelle
R représente un groupe alkyle,
éventuellement en présence d'un diluant ou éventuellement en présence d'un agent neutralisant les acides,
soit
b) avec des dérivés de dialkylformamide répondant à la formule 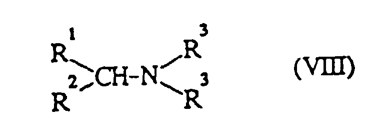 dans laquelle
R¹ et R² représentent, indépendamment l'un de l'autre, un groupe alcoxy ou un groupe dialkylamino, et
R³ représente un groupe alkyle,
éventuellement en présence d'un diluant, puis on fait réagir les dérivés d'acide 2-dialkylaminoacrylique ainsi obtenus répondant à la formule 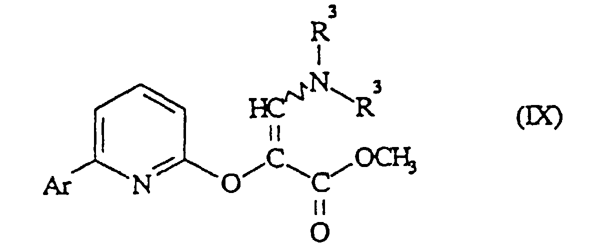 dans laquelle R³ et Ar ont la signification indiquée ci-dessus,
ultérieurement avec de l'eau, éventuellement en présence d'un acide,
et on fait réagir les dérivés d'acide acrylique obtenus dans lesquels E représente un atome d'hydrogène, éventuellement avec des hydroxydes de métaux alcalins en présence d'un diluant.

9. Esters pyridyloxyacétiques de formule (VI) 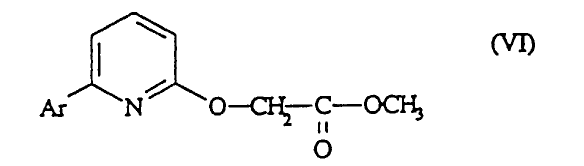 dans laquelle
Ar a la signification indiquée à la revendication 1.

10. Dérivés d'acide 2-dialkylaminoacrylique de formule 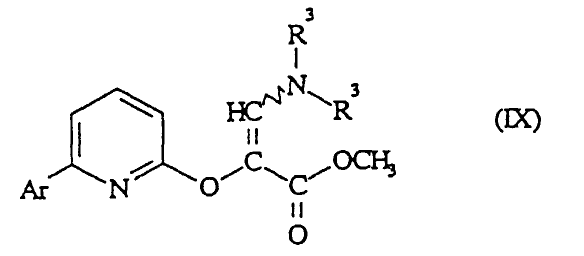 dans laquelle
Ar a la signification indiquée à la revendication 1 et
R³ représente un groupe alkyle.
